# EUROPEAN PATENT APPLICATION

(11) **EP 1 529 536 A1**
(43) Date of publication of application: **11.05.2005**
(21) Application number: 03292773.3
(22) Date of filing: 05.11.2003
(51) Int. Cl.: A61K 39/39, A61K 9/16, A61K 47/02, A61P 33/06

(54) **Immunogenic composition having improved immunostimulation capacity**

(71) Applicant: INSTITUT PASTEUR, 75724 Paris Cédex 15 (FR); CENTRE NATIONAL DE LA RECHERCHE SCIENTIFIQUE (CNRS), 75794 Paris Cedex 16 (FR)
(72) Inventor: Longacre, Shirley, 75006 Paris (FR)
(74) Representative: Desaix, Anne

(57) **Abstract**

The present invention relates to an immunogenic composition having an improved capacity for immuno-stimulation of the immune system of a host to whom it is administered. The present invention especially discloses a composition comprising, as active principle, molecules which would not, as such, elicit the immune system of a host, in a manner sufficient to derive a significant immune response to said active principle and especially to derive a response enabling the use of said composition as a vaccine composition. It relates more particularly to an immunogenic composition comprising a determined active principle and sucrose acetate isobutyrate (SAIB). These immunogenic compositions can also comprise solvents and additives such as bio-degradable polymers or adjuvants . The invention is especially useful for the preparation of immunogenic compositions wherein the active principle is a purified antigen, especially a synthetic or a recombinant antigen or polypeptide against which an immune response in a host is sought.

## Description

### Technical Field

The present invention relates to an immunogenic composition having an improved capacity for immuno-stimulation of the immune system of a host to whom it is administered. The present invention especially discloses a composition comprising, as active principle, molecules which would not, as such, elicit the immune system of a host, in a manner sufficient to derive a significant immune response to said active principle and especially to derive a response enabling the use of said composition as a vaccine composition. It relates more particularly to an immunogenic composition comprising a determined active principle and sucrose acetate isobutyrate (SAIB). These immunogenic compositions can also comprise solvents and additives such as bio-degradable polymers or adjuvants.

The invention is especially useful for the preparation of immunogenic compositions wherein the active principle is a purified antigen, especially a synthetic or a recombinant antigen or polypeptide against which an immune response in a host is sought. Because of the processes involved in their preparation, allowing especially a level of purity of the obtained product which is far higher than that of corresponding products purified from pathogens, synthetic or recombinant polypeptides or derivatives thereof indeed often require a particular formulation to enable their use as antigens for active principles in immunogenic compositions, especially require combination with compounds having immuno-stimulatory properties suitable to enhance the specific immunogenic reaction of the host receiving the composition.

The invention accordingly relates, in a preferred embodiment, to compositions useful for eliciting an immune response in a host, preferably a protective immune response against pathogens e.g., for vaccination of hosts against pathogens.

Hosts are either animal hosts, especially mammals, or humans.

In a particularly preferred embodiment, a composition of the invention enables elicitation of an immune response, following administration of only one or two doses of the composition i.e., under conditions that abrogate the need to carry out repeated administration of the immunogenic composition in order to obtain the desired effect.

A vaccine would be prepared from an immunogenic composition of the invention which would be easy to manufacture, stable at ambient temperatures to avoid the cold chain, easy to administer, efficient without the need to repeat administration, and would confer long, especially life-long immunity.

Such advantageous properties are important to improve the reliability of vaccination campaigns in terms of efficiency of the protection and costs of manufacture and distribution, allowing affordable treatment, particularly in developing countries. Repeat administration of vaccine compositions for instance has important disadvantages, since in many cases people who receive the first dose do not return for the second administration, leading to loss of effective antibody titers. However most vaccines require a primary immunization followed by two or more boosters for optimum response.

### Background Of The Invention

An immunogenic composition is a composition that causes the immune system of a host, to develop a specific immune response against the active principle of the administered composition. In a particular embodiment, the response enables protection against detrimental consequences of infection by a pathogenic organism, or more generally protection against adverse effects resulting from the presence of a foreign compound, organelle or organism in a host, thereby providing a vaccine composition.

One problem frequently encountered in the course of immunization, especially active immunization, is that the polypeptide-based active principles used in the immunogenic composition often have a low potency as antigen i.e., are not sufficiently immunogenic as such, to raise an antibody titer or a cellular response sufficient to provide a specific immune reaction in the host, or to maintain or increase the antibody titer or other immune response over extended time periods. Immunogenic compositions especially those based on the use of synthetic and recombinant polypeptides as antigens, vary in effectiveness and are often not sufficiently immunogenic. Therefore, most immunogenic e.g., vaccine compositions, require combination of their active principles with adjuvants to elicit or to enhance the immune response of the host, and also require a primary immunization followed by one or more boosters for optimum immune response.

An ideal adjuvant would be such that it could be easily manufactured and formulated to be highly stimulatory for the immune system of the host after one or two injections. Compounds currently used as vaccine delivery systems and as adjuvants for the stimulation of the immune system such as liposomes and microcapsules do not efficiently combine these two features. Several subunit and peptide vaccines have been prepared with liposomes as a vehicle and tested as liposome based vaccines. Despite higher antibody titers obtained than those obtained with vaccines without adjuvant, several disadvantages remain. Indeed, liposomes show instability at ambiant temperature, some degree of toxicity, have a variable (non reproductible) antigen release capacity, are inefficient in trapping antigen leading to waste of the antigen, can lose entrapped antigen during storage, and are not adaptable to traditional methods of vaccine preparation. Controlled release of antigens can be also achieved with microspheres (Men *et al.,* 1995). In spite of this high potential for the release of antigens, important difficulties have been noticed with vaccine formulations using microspheres. For example, these difficulties encompass a complex costly manufacture process not amenable to GMP conditions for clinical use, a low encapsulation efficiency, peptide inactivation during the encapsulation process and under the acidic conditions of microsphere decomposition, impediments in controlling the release, and low immunoadjuvant properties. The most widely used adjuvant for humans at present is aluminium hydroxide (alum). However, the use of alum adjuvant for immunization has several disadvantages such inducing inflammation and stimulating local production of granulomas (Men et al. 1995). Furthermore, alum has not suitable immuno-stimulatory properties with active principles having a low potency as antigens. Finally, alum-type vaccines require several repeat injections at appropriately timed intervals in order to achieve long-lasting immune response. CpG oligodeoxynucleotides (ODN) have been commonly used as an adjuvant, since CpG ODN induces a good demonstrated immune activation. Nevertheless several disadvantages remain such as direct effects of CpG ODN which are T cell independent and antigen non-specific. Saponins have also been used as adjuvants, in particular in admixture with cholesterol and phospholipids in order to create immune-stimulating complexes. QS-21 is a preferred saponins which can enhance humoral, cell mediated and mucosal immune responses (McCluskie et al. 2001). In spite of its immunogenic quality, QS-21 has been associated with some discomfort and acceptability difficulties in hosts. Vaccines have also been prepared with PPD (Purified Protein Derivative of tuberculin) as a carrier (Lachmann et al. 1986). PPD provide very powerful T-cell help without inducing antibody response against itself. Furthermore, small peptides such as hapten coupled to PPD are recognized by T-cells and can induce an anti-hapten immunogenic response. Nevertheless, PPD have several disadvantages encompassing a low humoral response and adjuvant function. Finally, montanide, which is comprised in a group of oil/surfactant based adjuvants, has shown to produce antibody levels equivalent to Freund's complete adjuvant. ISA 50 and ISA 70 montanide adjuvants induce less inflammatory response than Freund's adjuvant, which has however the same major component, i.e., mannide oleate. In spite of this wide use of montanide for humans vaccination, low immunoadjuvant properties have been observed when montanide is combined with synthetic or recombinant antigens. There exists thus a real need for the design of efficient immunoadjuvants suitable for formulation of immunogenic compositions.

The present invention pertains to an immunogenic composition using particular adjuvants as immuno-stimulatory components providing advantageous adjuvant formulations having improved properties with regard to known immuno-stimulatory systems.

### Summary of the invention

In this respect, the inventors have determined that compositions comprising sucrose acetate isobutyrate, exhibit immuno-stimulatory capacity and can be suitable for stimulating immune responses in a host against active principles with which it is combined.

The invention hence comprises the use of such compositions for the preparation of immunogenic compositions, advantageously of vaccine compositions and a method for administering these compositions in a manner that elicits or enhances a specific response of the immune system of the host.

### Detailed description of the invention

In the present application, the term "adjuvant" means any substance or compound capable of promoting or increasing the immune response against a determined active principle in an animal, including a mammal, or in a human host, (i.e., a specific immune response), when compared to the particular immune response obtained in the same conditions when administering said determined active principle alone. For purposes of illustration, adjuvant's effects can occur as a result of a modification of the activities of cells that are involved in eliciting and maintaining an immune response in a host, especially through a humoral response, or as a result of modifying the presentation of antigen to the immune system. The adjuvant properties of a compound may especially result from various modes of action, including delayed delivery of the antigen, improved ingestion of the antigen by antigen-presenting cells (APC), elicitation of co-stimulators (Janeway et al. immunobiology, 1999).

As used herein, the term "immunogenic" relates to a compound or composition which, when administered to a host, is capable of stimulating the host's immune response against said compound or composition. In a preferred embodiment, the immunogenic composition is such that it is regarded as a vaccine composition e.g., it is suitable to stimulate the immune system of the host in such a way as to protect said host against the detrimental consequences of pathogenic agents when the latter come into contact with said host. Preferably the vaccine composition of the invention elicits protection against a pathogenic agent, most preferably long-lasting protection. Pathogenic agents include, but are not limited to, pathogenic organisms, such as viruses, bacteria, parasites or compounds inducing or favouring toxic or detrimental reactions to emerge or spread in a host. Accordingly, in order to prepare a vaccine composition against a pathogenic agent, the active principle can comprise the pathogenic agent as such, or parts thereof including organelles of pathogenic organisms or their constituents, especially polypeptides including proteins or peptides, nucleic acids such as DNA or RNA or derivatives thereof, for example vaccines against rabies. The invention is of particular interest when the active principle i.e., the antigen, is a product, especially a polypeptide prepared using recombinant technology or chemical synthesis. In such a case the obtained product is often characterized by a high degree of purity, but usually shows a low immunogenic capacity. Vaccines are formulated and administered in order to induce prevention, amelioration or treatment of diseases, especially infectious diseases, or of their detrimental effects in a host.

The immune response advantageously corresponds to a humoral response and/or cellular response and especially results in the production of antibodies and/or activated lymphocytes against the specific active principle of the vaccine composition; therefore the active principle qualifies as an antigen in the context of the immunogenic composition of the invention.

Except otherwise stated "antigen" according to the invention encompasses any molecule used as active principle in an immunogenic composition or in a vaccine composition. Accordingly, antigen encompasses isolated natural molecules, recombinant and also synthetic molecules.

The expression "immuno-stimulation" or "immuno-stimulatory" refers to the capacity of a compound, to elicit or to enhance a response of a host through its immune system against the administered antigen.

"Recombinant polypeptide" or "recombinant antigen" refers to a polypeptide or an antigen whose expression is induced in a cell as a result of introduction into said cell of a nucleotide sequence encoding the polypeptide or the antigen, or as a result of inducing or increasing expression of a native nucleotide sequence for the purpose of recovering said polypeptide or said antigen in appropriate conditions to prepare said active principles. "Polypeptide" means herein any chain of amino acids, e.g., includes proteins or peptides, regardless of length or post-translational modification (such as glycosylation, phosphorylation lipo-conjugation, or addition of other moieties such as glycosyl phosphatidyl-inositol (GPI)).

"Synthetic antigens" or "synthetic polypeptide" are obtained for instance by chemical synthesis of their amino acid sequence. Conventional chemical methods of synthesis include, but are not limited to, Fmoc chemical synthesis, Boc chemical synthesis, the conventional method described by Merrifield *et al.,* 1963, or the methods described by Neimark and Briand, or Kings et al. Peptide synthesis in Fmoc or Boc chemistry, can be realised by using a multichannel peptide synthesizer as the ABI 433A peptide Synthetizer or the Pioneer Peptide Synthesizer, both commercialized by Applied Biosystems.

In a first aspect, the present invention relates to a composition formulated in a manner which enables immuno-stimulation against a determined antigen to be elicited or enhanced in a host, wherein said composition comprises a viscous, non-polymeric, non-water soluble, liquid adjuvant material and said determined antigen. The novel composition is suitable for *in vivo* administration.

Preferably, the viscous, non-polymeric, non-water soluble, liquid adjuvant is sucrose acetate isobutyrate (SAIB).

Sucrose acetate isobutyrate is the common name for alpha-D-glucopyranoside, O-acetyl-tris-O-(2-methyl-1-oxopropyl)-beta-D-fructofuranosyl, acetate tris-(2-methyl propanoate). SAIB is a clear, viscous (~100,000 cP at room temperature), thermally stable, practically odorless liquid that is slightly yellow in color. SAIB results from the reaction of sucrose with acetic anhydride and isobutyric anhydride in the presence of catalyst. The typical ratio of acetyl:isobutyryl substitution on the sucrose is 2:6, and the esterified product is purified by filtration and distillation. SAIB is used for a long time in foods, cosmetics and industry. As food additive, it is used in beverages that contain essential flavoring oils as a weighting agent or flavor emulsion stabilizer to prevent separation of essential oils. SAIB food-grade marketed by Eastman Chemical Company under the commercial name *Sustane,* has been approved for use as stabilizer of emulsions of flavoring oils or clouding agent in approximately 45 countries, including United States, European Union, India, Mexico, Japan. Studies conducted on SAIB food-grade since the early 1960s (Food and Chemical Toxicology, volume 36, number 2, February 1998) and Governmental Authorizations demonstrate that SAIB food grade is safe for human consumption at allowed use levels. In cosmetics, SAIB is used as tackifiers in lipstick and as fixatives. In industry, SAIB high volume resistivity and good thermal hydrolytic stability make it useful in surface coatings and electrical insulation. More recently, SAIB has been disclosed as a mean for the delivery and controlled release of drugs as described in the US patents US 5,747,058, US 5,968,542 and US 6,051,558. SAIB has a high viscosity, which can substantially be changed by heat treatment or addition of solvents. Furthermore, SAIB is compatible with a large number of solvents and various components.

SAIB is present in the composition in an amount which enables the desired effect i.e., the immuno-stimulation of the immune system of the host, to be achieved, and in an amount suitable for *in vivo* administration.

Typically, the composition according to the invention comprises SAIB in an amount in the range from about 99.5 % to about 10 % by weight, and more preferably from about 50% to about 70 % by weight, relative to the weight of the composition.

The antigen, comprised into the liquid composition suitable for immuno-stimulation in a host, is preferably a recombinant or a synthetic antigen.

The inventors have shown that recombinant or synthetic antigens which are obtained as extensively purified products are often not capable of inducing an immune response in a host. Hence, most compositions containing such purified antigen(s) will benefit from the use of adjuvants according to the invention to elicit a strong immune response and avoid multiple administrations to the host.

The present invention concerns accordingly the use of a recombinant antigen resulting from expression in cells of a DNA encoding a polypeptide of interest, especially a polypeptide identified from a pathogenic organism and having potential interest as an antigen. When said encoding DNA is transferred into cells for expression, the cells used for expression encompass bacteria, including *E. coli,* and eukaryotic cells such as yeast cells, insect cells, vertebrate cells, including mammalian cells, for example COS and CHO cells and plant cells.

The cells are cultivated in conditions enabling the polypeptides, especially the proteins or peptides to be produced and harvested. The obtained recombinant polypeptides, having antigen potency if necessary, after association with adjuvant are capable of eliciting or enhancing an immune response which is primarily a humoral response, but which can be also a cellular one such as CTL (Cytotoxic T Lymphocyte) response.

The prepared recombinant antigen, or alternatively synthetic antigen is used in combination with an adjuvant of the invention, for the manufacture of an immunogenic composition formulated to elicit or to enhance an immune response, and especially a strong and/or specific immune response directed to the antigen.

In a preferred embodiment, the antigen used in the compositions is a polypeptide identified as a parasite antigen which can protect against the agent of malaria, *Plasmodium,* especially a "blood stage antigen", preferably a recombinant antigen.

The term "blood stage" characterizes the stage during which patients infected with the malaria agent develop periodic fevers, chills and other signs of malaria, and the parasites are intra-erythrocytic or in the form of an extracellular merozoite.

The inventors have proven that an efficient immuno-stimulation in hosts can be obtained with antigens suitable for the design of vaccine compositions against malaria, when such antigens are included in the compositions of the invention comprising the adjuvant, especially a sucrose acetate isobutyrate based adjuvant.

Malaria is a protozoal disease transmitted by the Anopheles mosquito, caused by minute parasitic protozoa of the genus *Plasmodium,* which infects human and insect hosts alternatively. Despite vigorous efforts by national and international health agencies, malaria continues to be a leading cause of morbidity and mortality in tropical and subtropical areas of the world. It is estimated that there are 300-500 million cases of malaria reported per year worldwide.

The life cycle of the malaria parasite is complex, undergoing several stages of differentiation in two hosts i.e., mosquito and human. The two major etiological agents for human malaria are *Plasmodium falciparum,* which accounts for approximately 80% of cases, and *Plasmodium vivax.* In addition, *Plasmodium malariae* and *Plasmodium ovale* are infectious for humans. *Plasmodium* sporozoites are injected into the skin during a mosquito's blood meal and invade liver cells. The liver and sporozoites stages together are called the pre-erythrocytic stage. Each sporozoite develops into up to 40,000 merozoites that burst out of the liver cells and enter the blood stream, where said merozoites invade blood cells, thus initiating the erythrocytic phase involving repeated cycles of red blood cell invasion. Finally, some of the merozoites mature into sexual forms which are taken up by mosquito during a blood meal.

During the blood stage, *Plasmodium falciparum* parasites infect the erythrocyte and express parasite antigens on its surface. After maturation, the infected red blood cells burst, releasing extracellular merozoites, the surface antigens of which are exposed to the immune system. Therefore, malaria blood stage antigens, which appear to be exposed to the immune system during malaria infection, are potential vaccine candidates. An immunogenic composition containing blood stage antigens could mimic the immunity developed in people living in malaria endemic areas who no longer experience clinical symptoms, but still harbor blood stage parasites at reduced densities. This type of immunogenic composition with blood stage antigens might be the most appropriate form for children and adults living in endemic countries.

The inventors have especially designed an immunogenic composition which comprises, as the active principle, the MSP1p19 antigen from *Plasmodium falciparum* (PfMSP1p19), described in patent application EP0880588 and Chitarra *et al.,* 1999, which is a blood stage antigen regarded as a potential candidate for the preparation of a vaccine composition for protection against malaria. According to the invention, the malaria antigen is formulated with an adjuvant as defined above.

A homologue of the major *Plasmodium falciparum* merozoite surface protein 1, MSP1, of around 200 kDa is found at the merozoite surface in all species of *Plasmodium* studied to date, anchored at its C-terminus by a glycolipid glycosylphosphatidyl-inositol (GPI) moiety. The *Plasmodium falciparum* MSP1 precursor is processed in at least two proteolytic steps, leading first to a non-covalently associated complex of MSP1 derived peptides, including a membrane bound 42 kDa C-terminal moiety. During the secondary processing step, the 42 kDa fragment is further cleaved to the C-terminal GPI anchored protein, MSP1p19 (size 11 kDa or 96 amino acids) which remains on the merozoite surface during and after invasion of the erythrocyte. The polypeptide is rich in cysteines and folds into two intimately associated epidermal growth factor (EGF)-like domains, each with 3 disulfide bridges, indicating that this antigen has a conformationally complex structure, which would need to be correctly reproduced in recombinant polypeptides. Another very important property of MSP1p19 is that, unlike many highly polymorphic *Plasmodium* surface molecules, it displays very limited polymorphism in wild isolates of parasites from diverse geographical locations. Thus an immunogenic composition based on this antigen would be useful in a vaccination context as it would be expected to potentially protect very well against most, if not all, heterologous parasite strains found in any endemic region.

In another particular embodiment of the invention, other antigens, especially blood stage antigens of malaria agents, are proposed including AMA-1, EBA-175, EMP-1, GLURP, MSP-1, MSP-2, MSP-3, MSP-4, MSP-5, Pf35, Pf55, RAP-1, RAP-2, RESA, SERA of *Plasmodium falciparum.* These antigens have been described in Path: Program for Appropriate Technology in Health (http://www.malariavaccine.org/mal-vac2-challenge.htm). Specifically, recombinant polypeptides produced in the baculovirus expression system as for example merozoite surface proteins 4 and 5 (MSP4 and MSP5) from *Plasmodium falciparum* have a strong potential interest in a vaccination context. The corresponding homologous antigens which exist in other *Plasmodium* species infecting human, such as *P. vivax, P. malariae, P. ovale,* would also be another embodiment of the invention as for example MSP1p19, Duffy Binding Ligand region II (DBL), CSP-1, MSP4 and MSP5 from *Plasmodium vivax.* Antigens from other stages of the parasite cycle as LSA-1 (EP0343186, EP0570489), LSA-3 (EP0343186, EP0570489, EP0833917), EXP-1 (Gunther *et al.,* 1991), RSP-1 and RSP-2 (Pouvelle *et al.,* 2000) would also be ideal for use in the immunogenic compositions of the invention.
Antigens from other microorganisms than *Plasmodium* can also be used in the composition of the invention, such as antigens from *Mycobacterium* species and more particularly from *Mycobacterium tuberculosis,* as for example ESAT-6 (Harboe *et al.,* 1996) or CFP-10 (Berthet *et al.,* 1998), antigens from HIV such as those described in EP 0201540, antigens from *Helicobacter pylori* such as those described in US 5,986,051 or antigens from HBV such as those described in US 5,314,808.
In addition, any of the above mentioned antigens could be produced in the baculovirus expression system with a C-terminal GPI modification or C- or N-terminal hydrophobic amino acid sequence to enhance immunogenicity.

For the purpose of the invention, an antigen can be used in immunogenic compositions of the invention, either alone or in combinations with other antigens.

In another particular embodiment of the invention, an antigen can be used in immunogenic compositions of the invention in freeze-dried form. The freeze-dried antigens are under advantageous form to allow manufacture and distribution of vaccine compositions through developing countries. Indeed, the freeze-dried form of antigen allows to avoid the cold chain as far as the distribution is easier.
Furthermore, addition of the above freeze-dried antigens to the immunogenic composition of the invention does not induce a dilution of others components of said composition. Accordingly, the concentration of the viscous, non-polymeric, non-water soluble liquid adjuvant is constant after addition of the freeze-dried antigen to the immunogenic composition. Absence of dilution effect would be interesting in particular for preparation and administration of vaccine compositions during vaccination campaigns.

In a preferred embodiment, the antigen used in the composition is an antigen soluble in the viscous, non-polymeric, non-water soluble liquid adjuvant of the invention, especially the sucrose acetate isobutyrate (SAIB). The SAIB-soluble antigen of the invention indeed does not provide an antigen precipitate during the preparation of the composition. Accordingly, the antigen solubility in SAIB is suitable for an easy manufacture and administration of the immunogenic composition.

The antigen is added in the composition of the present application in an effective dose to elicit an immunogenic response in the host. As used herein, a dose effective to elicit an immune response is one that causes antibody titers to increase compared to untreated animals or individuals. Antigen is typically present in the composition of the present invention in an amount in the range from about 0.1 µg and 5000 µg per composition dose, preferably about 0.1 µg and 1000 µg and more preferably about 0.1 µg and 100 µg.

When a potent antigen is combined and formulated with the adjuvant, the immunostimulatory property of the adjuvant may allow the use of lower doses of antigen or the use of the prepared composition in a manner which avoids multiple administrations to a host.

The proposed composition of the invention indeed provides the advantageous property that the minimum effective amount of antigen present as active principle can be used for the preparation of the composition when compared with the dose of antigen which would be involved in common immunogenic composition devoid of the viscous, non polymeric, non water soluble, liquid adjuvant and/or that the administration protocol may be designed to limit the number of administered doses, advantageously to enable that only one or two doses are administered to obtain an efficient long lasting immune response in a host.

In another embodiment of the invention, the composition comprises a viscous, non-polymeric, non-water soluble liquid adjuvant material, an antigen, and a solvent, which when added to the said liquid adjuvant material, provides a lower viscosity liquid composition suitable for administration.

The high viscosity liquid adjuvant material significantly decreases in viscosity when mixed with the solvent. Therefore the solvent is added in the composition in an amount permitting to form a composition having a desired viscosity that renders it appropriate for *in vivo* administration to animals, including but not limited to mammals, and especially to humans. The solvent should be chosen according to its intended use *in vivo* and be compatible with the constraints of the manufacture of an immunogenic composition.

Therefore, the mixture may comprise one or more solvents selected from the group consisting of ethanol, ethyl lactate, propylene carbonate, glycofurol, N-methylpyrrolidone (NMP), 2-pyrrolidone, propylene glycol, acetone, methyl acetate, ethyl acetate, methyl ethyl ketone, benzyl alcohol, benzyl benzoate, benzoyl benzoate, triacetin, dimethylformamide, dimethylsulfoxide, tetrahydrofuran, aprolactam, decylmethylsulfoxide, oleic acid, triglyceride and 1-dodecylazacycloheptan-2-one. In the present application, the preferred solvents are ethanol, N-methylpyrrolidone (NMP), and benzoyl benzoate.

In a preferred embodiment, the present solvent is in an amount from about 5% to about 55% by weight and more specifically from about 30% to 50% by weight, relative to the weight of the composition.

The composition described herein can optionally also comprise further additives in order to resolve a variety of problems encountered in the course of immunization using synthetic or recombinant antigens : increase the immunogenicity of antigen (improve the level of antibody titers), favor the induction of certain antibody isotypes or certain cellular responses, providing an improved immune protection of the host. In an advantageous embodiment, one category of additives according to the present invention are bio-degradable and bio-absorbable polymers. Langer et al. (Nature, 1998 vol. 392 supp) propose incorporation of immune adjuvants into polymers (vaccine delivery system, VDS) so that when the polymer degrades and the vaccine principle is slowly released, an adjuvant is also released that stimulates the immune response further.

These polymers may be synthesized for example via the ring-opening polymerisation of cyclic dimer of lactide or glycolide and may be selected among those used in clinical domain like suture, drug release systems. These additives are particularly used as major components in microsphere technology and have effects on the rate of drug release. For purposes of illustration, bio-degradable and bio-absorbable polymers are selected from the group consisting of poly(lactide), poly(lactide-coglycolide), poly(glycolide), poly(caprolactone), polyamides, polyanhydrides, polyamino acids, polyorthoesters, polycyanoacrylates, poly(phosphazines), poly(phosphoesters), polyesteramides, polydioxanones, polyacetals, polyketals, polycarbonates, polyorthocarbonates, degradable polyurethanes, polydroxybuty-ates, polyhydroxyvalerates, polyalkylene oxalates, polyalkylene succinates, poly(malic acic), chitin, chitosan, terpolymersoxidized cellulose and copolymers. In the most advantageous embodiment, the immunogenic composition according to the present invention comprises poly(DL-lactide) of the poly(lactide) additive (DLPL).

Additional adjuvants, a further category of additives, can be also added in the liquid composition of the invention. Non-limiting examples of adjuvants include Alum, CpG DNA or various cytokines, in amounts necessary to enhance the immune response in a host. Those skilled in the art will decide for each case the appropriate supplementary adjuvants.

The invention also concerns an immunogenic composition comprising sucrose acetate isobutyrate and N-methylpyrrolidone in a ratio of about 70:30 by weight, about 10% DLPL and the antigen, for example 20µg of PfMSP1p19 recombinant antigen. This mixture is one preferred composition of the present invention.

The compositions described above can be administered to the host topically, systemically or/and parenterally. More specifically, the compositions according to the invention enable systemic administration consisting in mucosal, oral, rectal, vaginal or nasal administration. The immunogenic compositions can also be administered by parenteral administration consisting in intravenous, subcutaneous, intramuscular, intradermal or intraperitoneal administration. The skilled person in this field will decide for each case the suitable way of delivery according to the result to be achieved.

The invention also concerns compositions of the invention which are formulated to be suitable for eliciting or enhancing a specific immuno-protection in a host after the first injection.

The inventors have also observed that the composition of the invention can be formulated in a manner which induces a long-lasting immunity from the first injection for some antigens. Indeed, the composition induces a good immune response long after the first injection, eliciting an antibody titer which may be sufficient to maintain protection over a long period of time, advantageously a life-long protection. This immune response is even surprisingly long-lasting when using poorly immunogenic antigens such as recombinant antigens, or when compared to the lower immunogenic response obtained with compositions containing recombinant antigens and adjuvants such as Alum. These results emphasize the importance of the choice of the adjuvant for efficiently eliciting antibodies or cellular responses especially directed against a poorly immunogenic antigen.

The inventors have observed that compositions according to the present invention afford a sustained immune response of the host, after administration of a single dose, and especially an immune response lasting as long as 33 weeks.

The invention also relates to an adjuvant kit comprising the viscous, non-polymeric, non-water soluble liquid adjuvant material, the determined antigen and one or several solvents described above which when added to the viscous non-polymeric non-water soluble liquid adjuvant material provides a low viscosity liquid composition. Optionally, said kit comprises additives as defined herein. It can also comprise directives for administration of the obtained composition, in order to carry out a vaccination protocol.

The invention also concerns a method of immunization wherein the viscous, non-polymeric, non-water soluble liquid adjuvant material as immuno-stimulator, is administered before, simultaneously with or after the administration of a determined antigen, at the same site as the antigen, to elicit or to enhance an efficient immune response in a host. The above method of immunization is advantageously suitable for eliciting or enhancing a specific immuno-protection in a host after the first injection. Preferably, SAIB, formulated and administered as an adjuvant, is mixed with solvent(s) in an amount which is suitable for *in vivo* applications. Therefore, it does not necessarily appear to be critical in the vaccination protocol included in the kit whether SAIB is injected before, after or simultaneously with the antigen, even though conventionally the adjuvant and the antigen are simultaneously administered.

Finally, in another specific embodiment, the present invention relates to a use of the viscous non-polymeric non-water soluble liquid adjuvant material as immuno-stimulator for the manufacture of a composition suitable for eliciting or enhancing immuno-stimulation in a vaccine. Preferably, the present use of the adjuvant material concerns the manufacture of an immunogenic composition which comprises a recombinant or synthetic antigen as active principle and more specifically the manufacture of an immunogenic composition against malaria pathogens. In a preferred embodiment, the present use of the adjuvant material concerns the manufacture of a vaccine which comprises at least a recombinant or synthetic antigen as active principle and more specifically the manufacture of a vaccine against malaria pathogens.

### Description of the drawings

Figure 1 shows the antibody response to PfMSP1p19 induced by SBS1, SBS2, SBS3, SBS4, SBS5 and SBS6 compositions [Figures 1 (a) to (f)] as measured by ELISA technique 3.5 weeks after injection.
Figure 2 shows the antibody response to PfMSP1p19 induced by SBS1, SBS2, SBS3, SBS4, SBS5 and SBS7 compositions [Figures 2 (a) to (f)] as measured by ELISA technique 9.5 weeks after injection.
Figure 3 shows the antibody response to PfMSP1p19 induced by SBS1, SBS2, SBS3, SBS4 and SBS5 compositions [Figures 3 (a) to (e)] as measured by ELISA technique 15 weeks after injection.
Figure 4 shows the antibody response to PfMSP1p19 induced by SBS1, SBS2, SBS3, SBS4 and SBS5 compositions [Figures 4 (a) to (e)] as measured by ELISA technique 22 weeks after injection.
Figure 5 shows the antibody response to PfMSP1p19 induced by SBS3 as measured by ELISA technique 27 weeks (Figure 5a) and 33 weeks (Figure 5b) after injection.
Figure 6 shows the antibody response to PfMSP1p19 induced by the following compositions:
   - Montanide and PfMSP1p19 antigen as measured by ELISA technique 3.5 weeks after injection (Figure 6 a);
   - Alum and PfMSP1p19 antigen as measured by ELISA technique 3.5 weeks after injection (Figure 6 b).

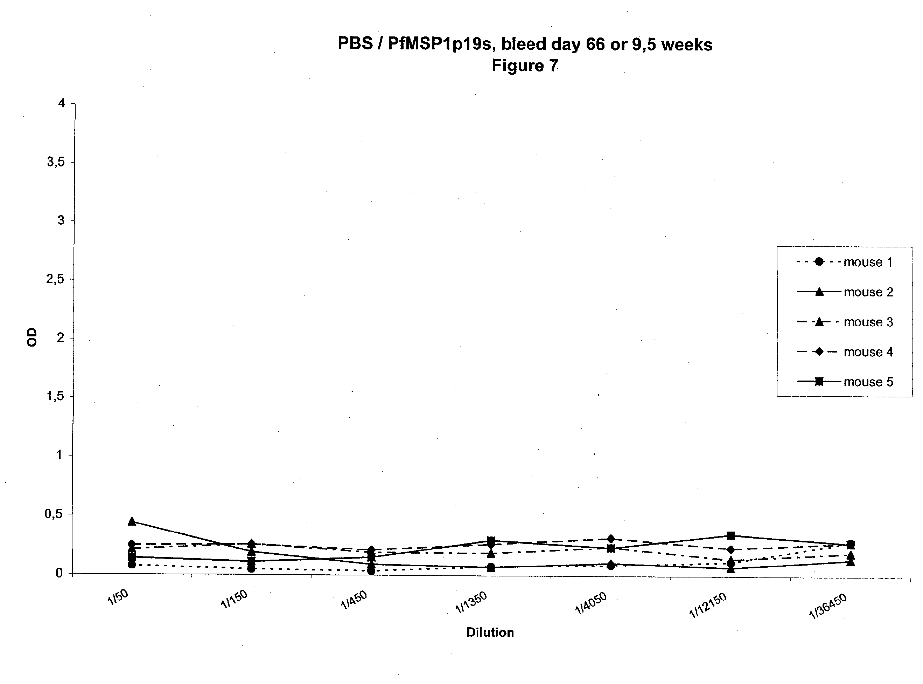

Figure 7 shows the antibody response to PfMSP1p19 induced by a composition only containing PfMSP1p19 antigen in phosphate buffer salt (PBS).

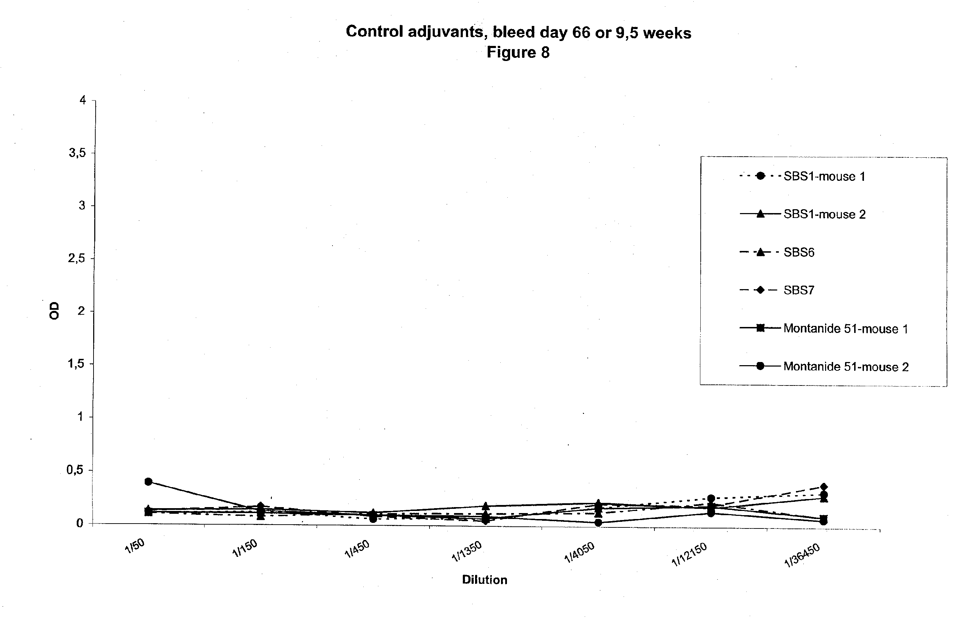

Figure 8 shows the antibody response to PfMSP1p19 induced by four control compositions that is to say adjuvant formulations without any antigen.

### Experimental

Below, the present invention will be described by way of examples, which are only to be construed as illustrating the invention and not limiting the scope thereof as defined by the appended claims. All references below and elsewhere in the present application are hereby included herein by reference.

### 1. Animals

Immunogenic compositions are tested using OF1 mice as experimental animals. OF1 mice were chosen for testing the immunogenic compositions because they are outbred mice with many diverse histocompatibility genes, and therefore considered to be a good model for diverse human populations. The specific OF1 mice used in the evaluation of the compositions were obtained from IFA Credo. The animals included in the evaluation of the compositions were 8 week-old females.

### 2. Blood samples

Mice were left for about one minute under an infra-red lamp to stimulate blood flow to the extremities. The vein on the ventral side of tail was cut with a scalpel and 200 to 500 µl of blood were collected into an eppendorf tube. Blood samples were left 16-24 hours at 4°C or 2 hours at 37°C. Afterward, the blood samples were centrifuged 10 minutes at 15,000 rpm in Sigma 2MK tabletop centrifuge. Serum, supernatant layer, was removed and kept at 4°C less than 1 week until tittered and thereafter at - 20°C.

### 3. Virus antigen preparation

Insect cells are the preferred hosts for the baculovirus vectors of the invention. In the present invention, the preferred hosts is Sf9 and High 5 insect cells. Therefore, Sf9 or High 5 insect cells were infected with recombinant baculovirus containing the gene sequence for *Plasmodium. falciparum* MSP1p19 (PfMSP1p19). Said gene sequence comprised the following elements: (i) 93 residues corresponding to the *bona fide* p19 segment (Uganda-Palo Alto allele) minus the 21 most C-terminal residues mediating GPI anchor transformation, and thus allowing secretion of a soluble product; (ii) 6 C-terminal histidine for metalloaffinity purification; (iii) 16 residues upstream from the p19 fragment to provide a more complete analogue of the p19 proteolytic site; (iv) 2 residues derived from the EcoRI site used to make this particular construction; (v) 43 N-terminal MSP1 residues containing the signal sequence and N-terminal residues.
Infected cells were incubated for 2 to 3 days at 27°C in suspension cultures either in spinner flasks or in an air-lift fermentor (Serono Pharmaceutical Research Institute, Geneva, Switzerland). Then, the culture supernatants were dialysed before metalloaffinity chromatography (Talon or Ni-NTA resin). Fractions eluted with 100 or 500 mM imidazole and containing PfMSP1p19 as determined by SDS-PAGE and Coomassie blue staining were pooled, dialysed against 50 mM ammonium carbonate, and lyophilised in aliquots of 100 or 400 µg. Alternatively pooled metalloaffinity fractions were subjected to Q-Sepharose chromatography and fractions containing PfMSP1p19 were pooled, dialysed and freeze-dried as above (Serono Pharmaceutical Research Institute, Geneva, Switzerland).

### 4. Formulation of compositions

### Tested compositions :

Seven compositions were prepared, containing equal amounts of lyophilized *Plasmodium falciparum* MSP1p19 baculovirus recombinant antigen (20 µg) mixed with seven different SBS adjuvant solutions composed of sucrose acetate isobutyrate (SAIB) and having the same final volume (200 µl) per immunogenic composition dose.
SBS1 composition containing 70:30 SAIB / ethanol.
SBS2 composition containing 70:30 SAIB / NMP.
SBS3 composition containing 70:30 SAIB / NMP w/10% DLPL.
SBS4 composition containing 70:30 SAIB / benzyl benzoate.
SBS5 composition containing 50:50 SAIB / benzoyl benzoate.
SBS 6 composition containing 60:40 SAIB / NMP w/10% DLPL.
SBS 7 composition containing 60:40 SAIB / benzoyl benzoate.

### Control compositions :

Four compositions were prepared, which did not contain PfMSP1p19 baculovirus recombinant antigen but had the same final volume (200 µl) per dose consisting only of adjuvant solution.
SBS 1 composition containing 70:30 SAIB/ethanol.
SBS 6 composition containing 60:40 SAIB / NMP w/10% DLPL.
SBS 7 composition containing 60:40 SAIB / benzoyl benzoate.
Montanide ISA51.

Three other control compositions were prepared, which contained equal amounts of PfMSP1p19 baculovirus recombinant antigen (20 µg) and had the same final volume (200 µl) per dose.
Montanide ISA51 and PfMSP1p19 antigen.
Alum and PfMSP1p19 antigen.
Phosphate buffer saline (PBS) and PfMSP1p19 antigen.

### 5. Protocols of immunogenic treatments

### Tested compositions :

The animals were divided into seven groups of five animals according to the experiment and were kept isolated during the evaluation of the immunogenic compositions. The mice were injected subcutaneously with 200 µl of one of the described above compositions. The injections were given once without booster injection in the next weeks. Blood samples were taken after primary injection i.e., 3.5 weeks (day 23) after injection, 9.5 weeks (day 66) after injection, 15 weeks (day 105) after injection, 22 weeks (day 147) after injection, 27 weeks (day 183) after injection and 33 weeks (day 268) after injection.

### Control compositions :

For comparison, the experiment also included control groups. The animals were divided into seven groups of five animals according to the experiment and were kept isolated during the evaluation of the immunogenic compositions. The control groups were injected subcutaneously with 200 µl of the compositions described above. The injections were given once without booster injection. Blood samples were taken after 3.5 weeks and 9.5 weeks after the primary injection.

### 6. Serological test: ELISA test

### Reagents for ELISA :

ELISA coating buffer was PBS.
Wash buffer was 0.1% Tween 20 in PBS.
Blocking buffer was 0.5 % gelatin in PBS
Dilution buffer was 0.1% Tween 20 and 0.5 % gelatin in PBS.
OPD substrate was a 2 mg OPD tablet (Dako) dissolved in 5 ml of 0.03 % hydrogen peroxide.

### Coating of microtiter plates:

PfMSP1p19 diluted in phosphate buffered saline (PBS) (0.5 µg/ml; 100 pl/well) was coated onto 96-well Nunc-Immuno plates with maxiSorp surface. Plates were stacked, covered with an adhesive plastic film and left 16-24 hours at 4°C or 2 hours at 37°C.

### Washing and blocking plates:

The plates were washed 3 times in wash buffer avoiding air bubbles in wells, and were emptied with vigorous tapping between washes. Then, each well of the plates was saturated with 100 µl of blocking buffer. The plates were incubated 1 hour at 37°C and finally emptied.
Sera dilutions and antibody reaction with coated antigen:
Serum samples were diluted 1:50 in dilution buffer. Aliquots of 100 µl of dilution buffer were placed into all wells except those in row n°2. Row n°1 of the microtiter plates was negative control containing 100 µl of buffer dilution only. Row n°2 of the microtiter plates was duplicated and containing 150 µl of 1:50 sera dilutions. Fifty µl from each well in row n°2 was removed and added to corresponding wells in row n°3 then mixed by pipetting up and down 10 times. It was continued in this manner adding consecutively 50 µl from each well in row n°3 to wells in row n°4 etc, and 50 µl was removed from each well in the last row. Therefore, sera dilutions in rows 2 to 8 corresponded to 1:50, 1:150, 1:450, 1:1350, 1:4050, 1:12150 and 1:36450. The plates were incubated 1.5 hours at 37°C then they were washed 3 times with wash buffer.

### Antibody conjugate reaction :

Goat anti-mouse IgG conjugated with peroxidase (Bio-Rad) was diluted 1:1000 in dilution buffer. Aliquots of 100 µl of diluted antibody were added to each well. The plates were incubated 1 hour at 37°C and washed 3 times with wash buffer.

### Peroxidase substrate reaction :

Hundred µl of OPD substrate was added to each well. The plates were incubated for 10 minutes at room temperature, i.e. 20°C, in the dark then 50 µl of 3N HCl was added in each well of said plates. Optical density (OD) was read immediately at 490 nm and 650 nm.

### 7. Safety of the immunogenic compositions

The mice treated by immunogenic compositions of the present invention were observed daily for each bleeding. All animals used for testing the immunogenic compositions within SAIB composition were remained clinically healthy during the entire period of the experiments. Neither local reactions nor alterations in the animals behaviour were observed, and apparently the mice developed normally. However, one death was apparently unrelated to the experimental treatment.

### 8. Results

### Comparison of several adjuvants with the recombinant PfMSP1 p19 antigen

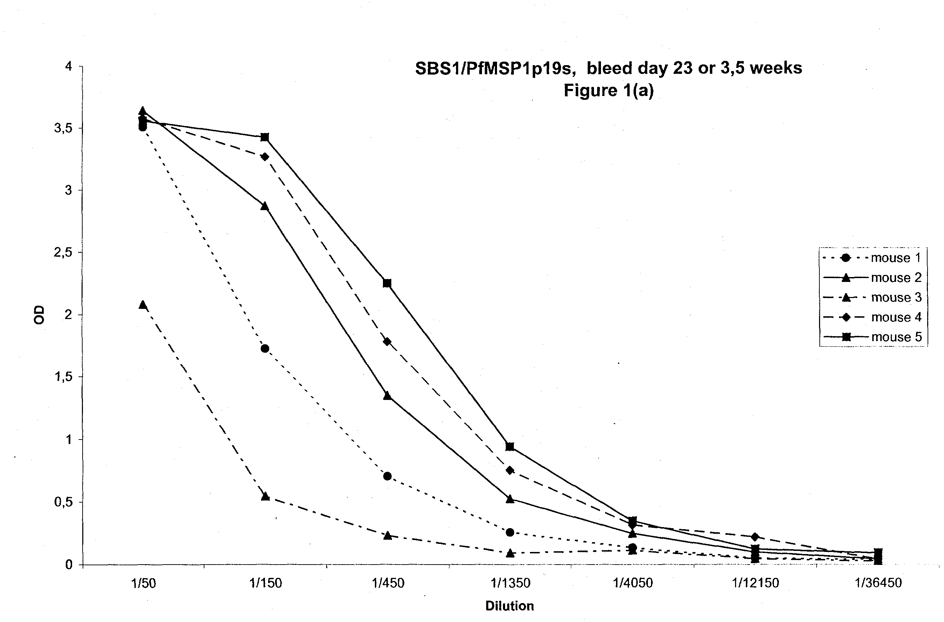

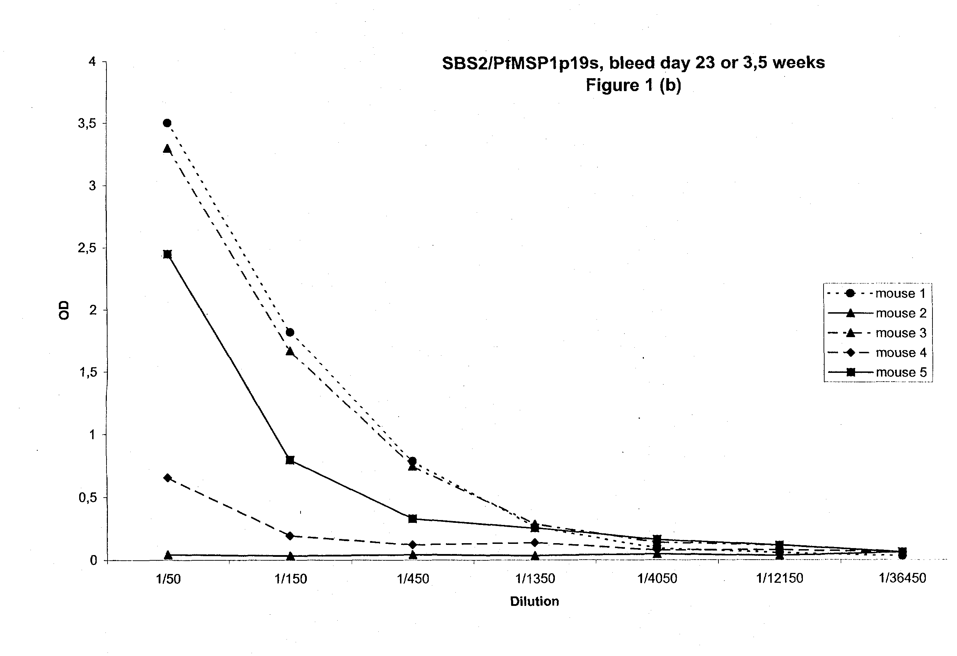

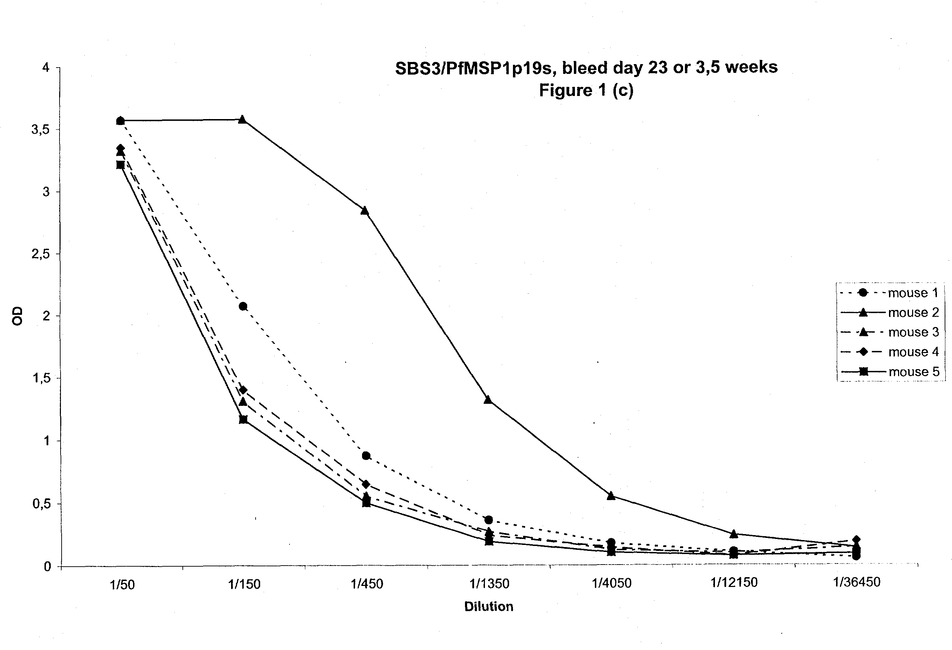

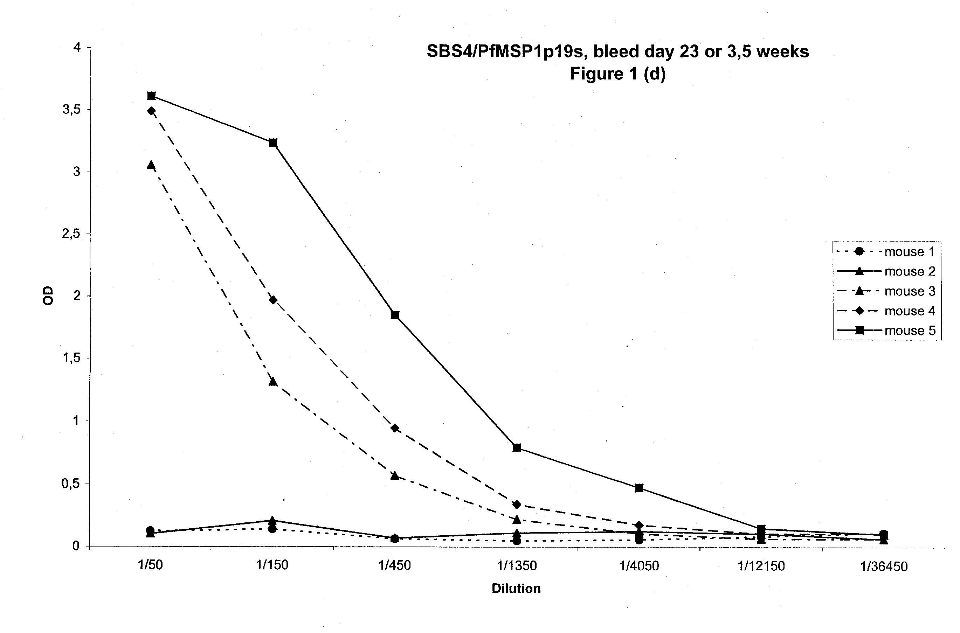

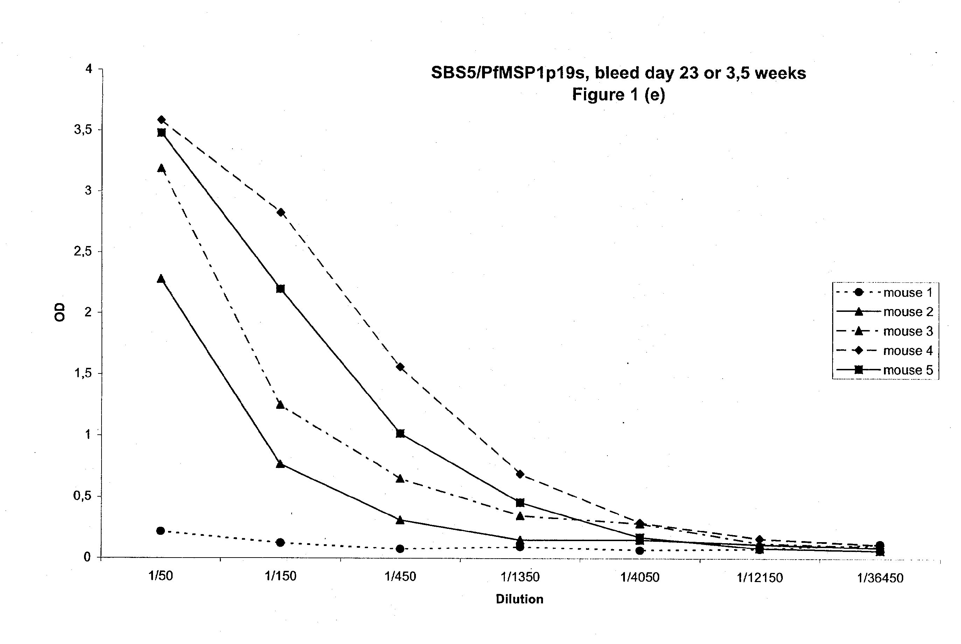

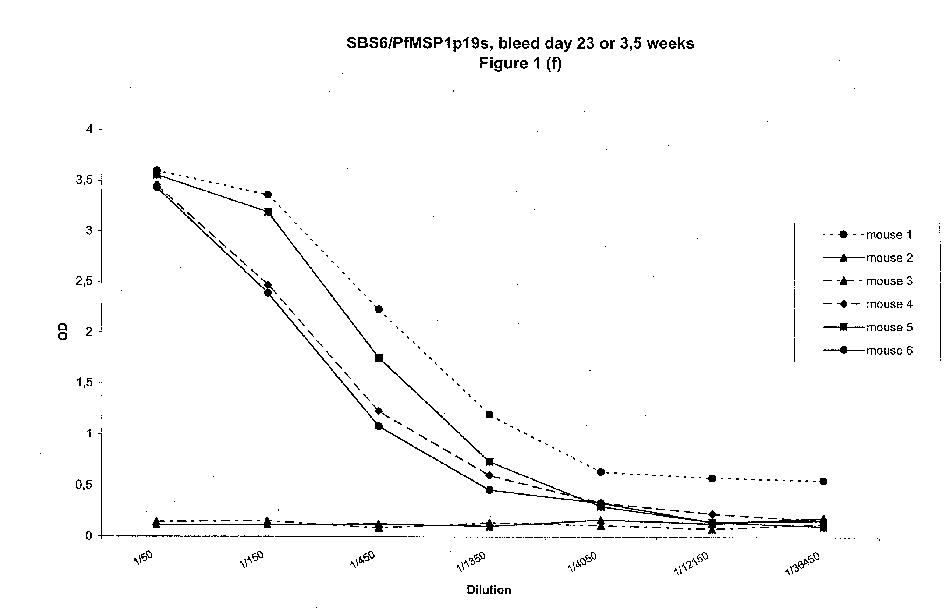

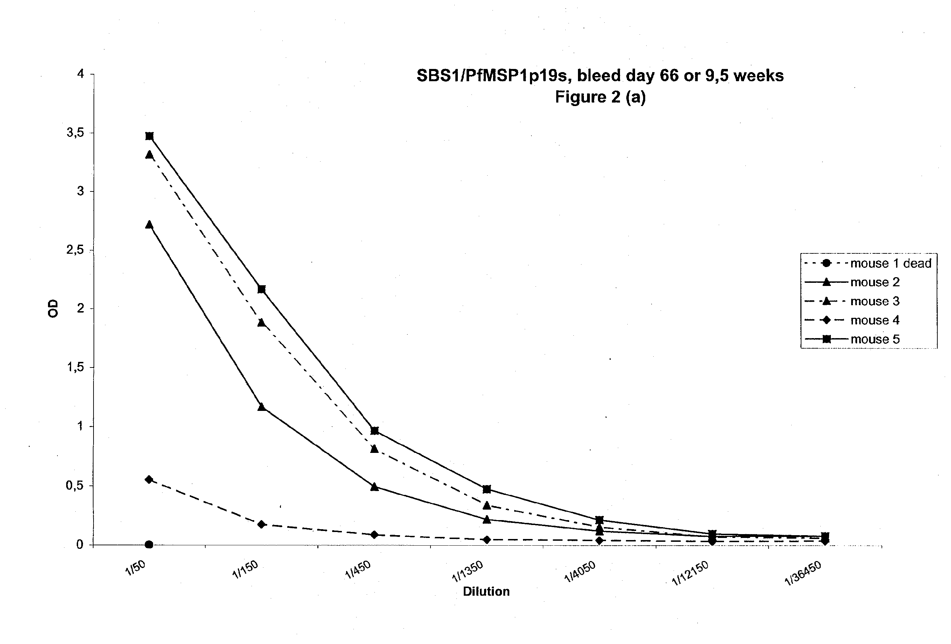

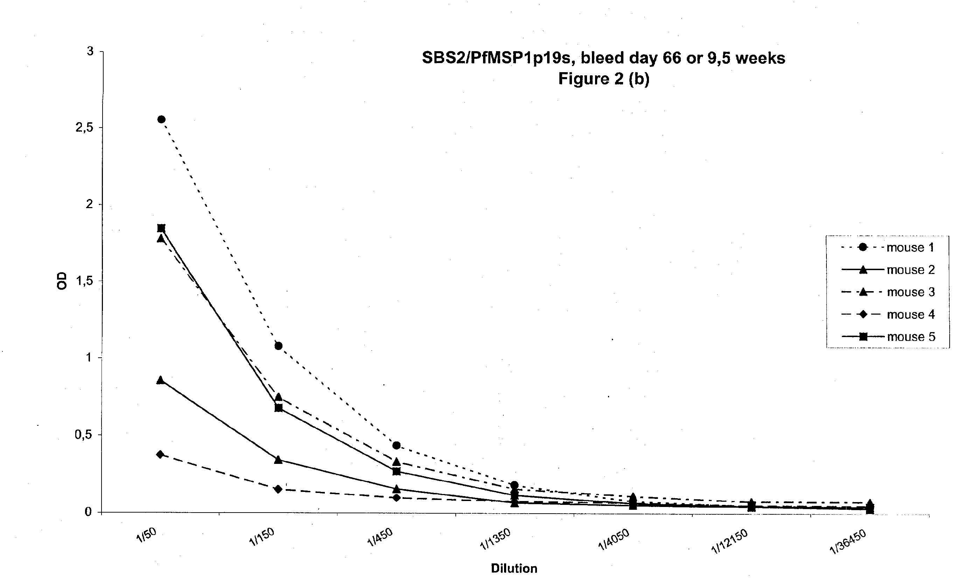

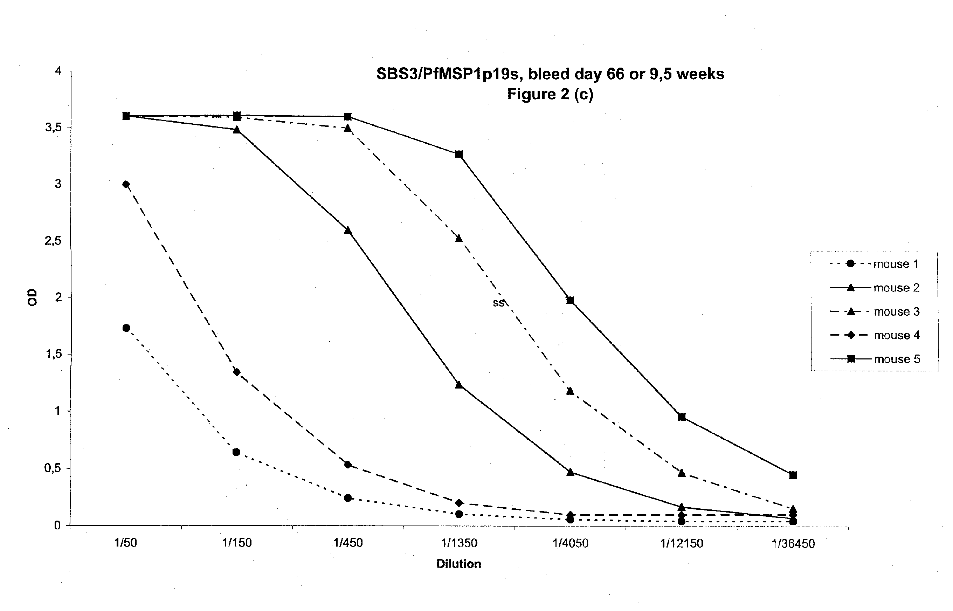

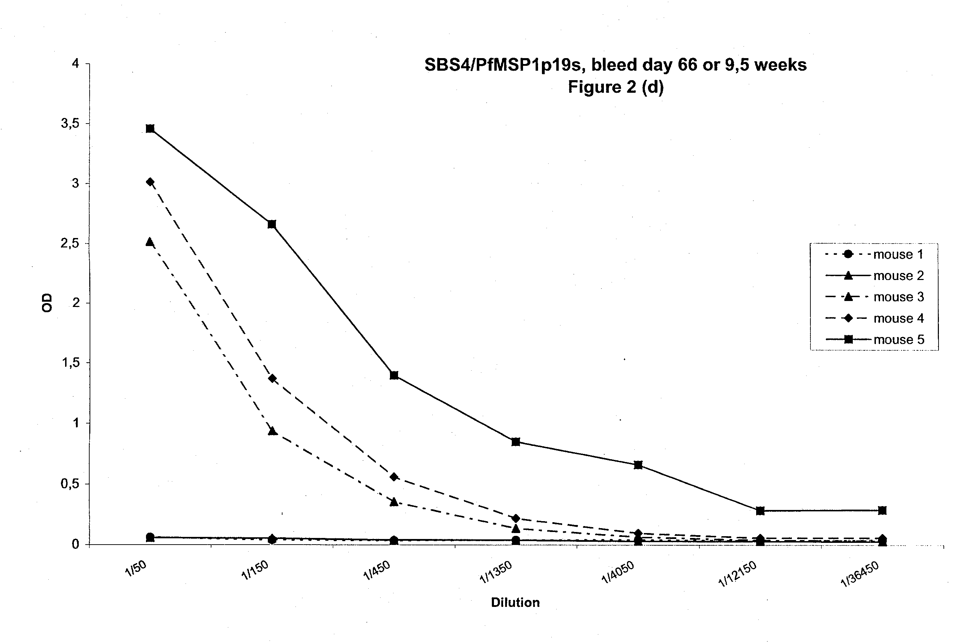

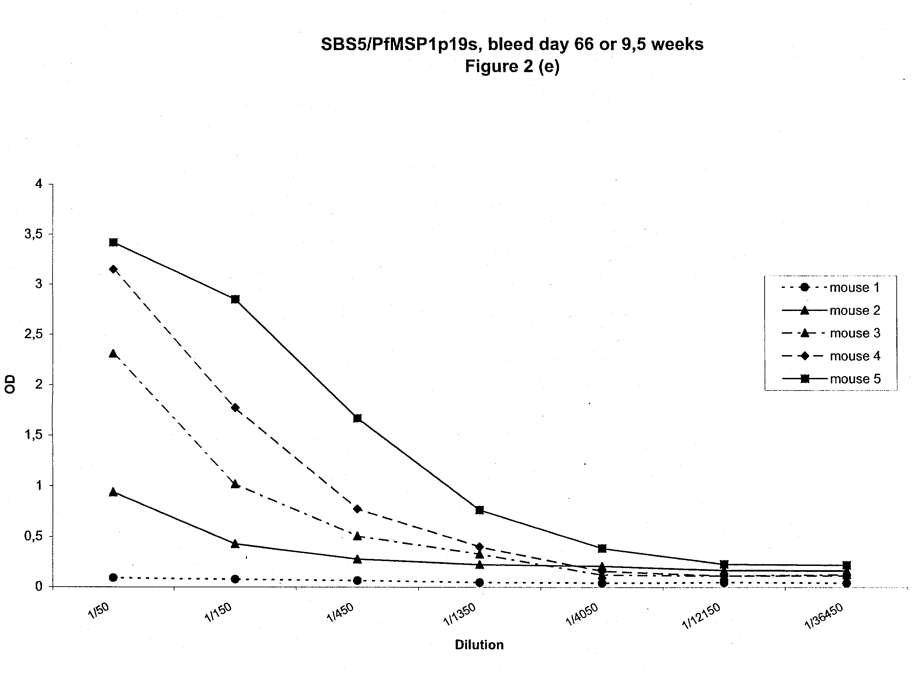

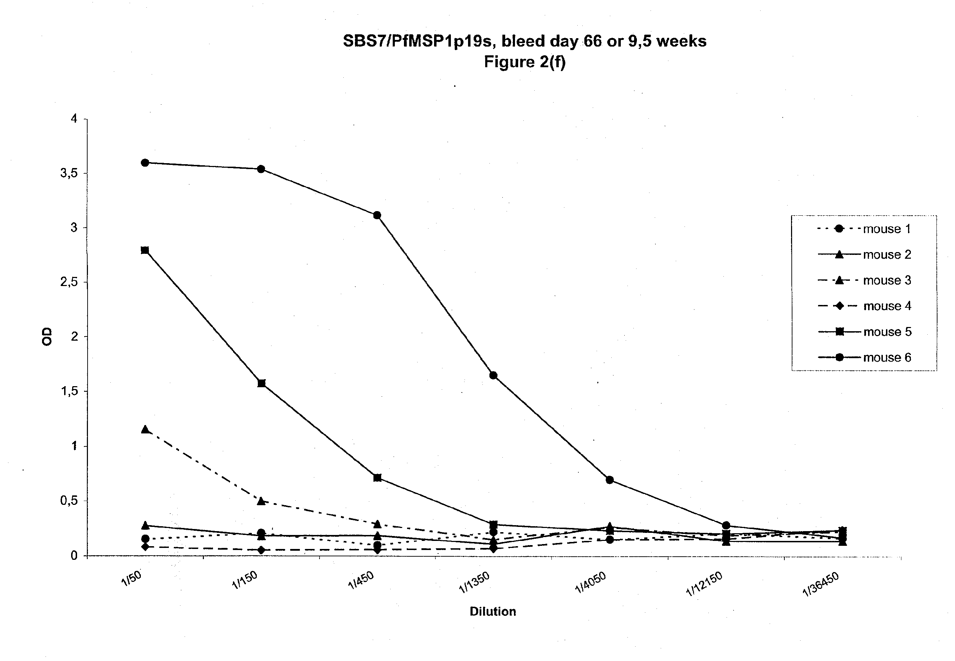

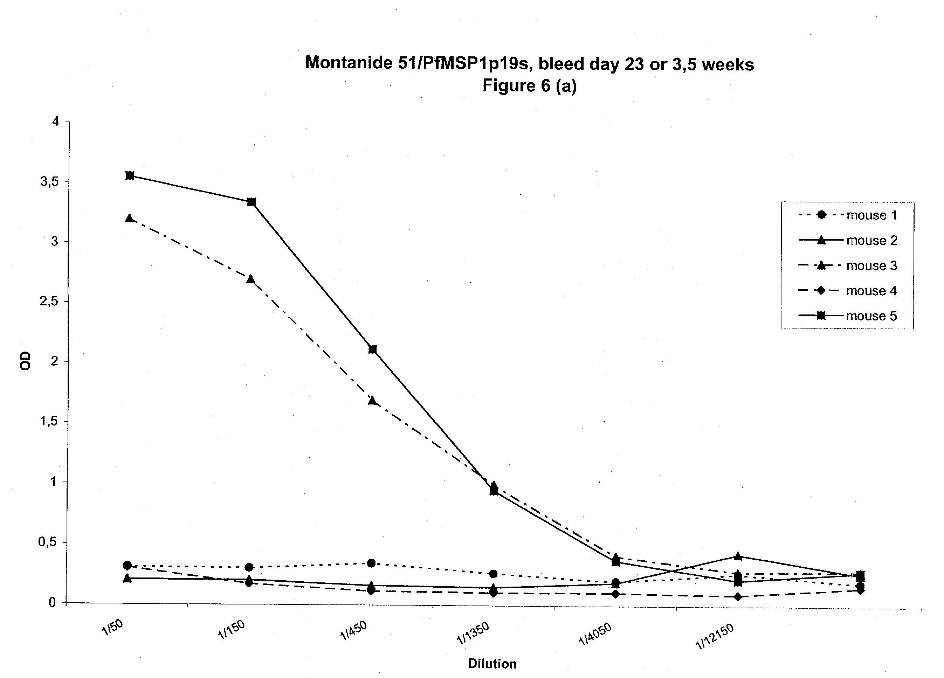

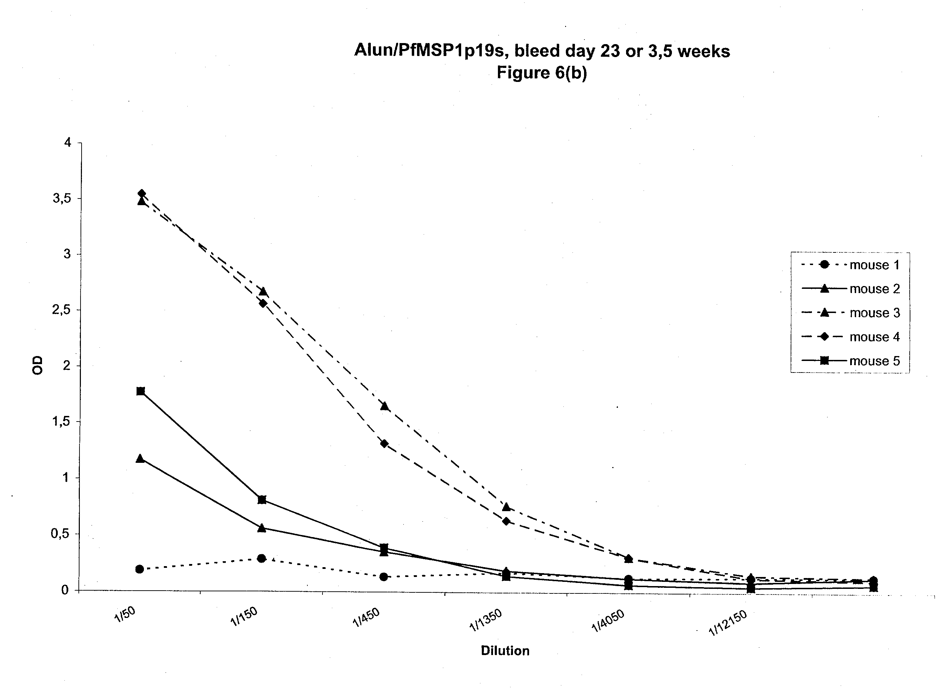

The results presented in Figure 6 show the values of antibody titer recorded 3.5 weeks after the primary injection, for the sera from animals immunized with the composition consisting in PfMSP1p19 recombinant antigen and alum or Montanide ISA51 adjuvants. The antibody response was determined by means of the ELISA test. Antibody titers, recorded from animals immunized with the composition consisting of PfMSP1p19 antigen and Montanide ISA51, are quite heterogeneous with 3/5 animals showing little or no response as early as 3.5 weeks after the primary injection. Thus, Montanide ISA51 is not as good as adjuvant to induce an immune response directed against a recombinant antigen, which is poorly immunogenic. In the same way, low antibody titers were observed 3.5 weeks after the primary injection with an immunogenic composition consisting of PfMSP1p19 recombinant antigen and Alum. 9.5 weeks after the primary injection with the Montanide ISA51 and Alum compositions, the antibody titers were very low (data not showed). Thus a classical adjuvant suitable for human use is not able to elicit a good immune stimulation in a host against a non-immunogenic antigen from the first injection. Indeed, the antibody titers were equal or clearly lower than the values recorded 3.5 and even about 9.5 weeks after the primary injection (Figures 1 and 2) for the sera from mice injected with compositions including SAIB. Therefore, according to the present invention, as unexpected, it has been shown that the use of SAIB in an immunogenic composition surprisingly induces higher and more long-lasting antibody titers than common adjuvants. The time period of an immune response in mice is increased at least four-fold with immunogenic compositions of the invention rather than immunogenic compositions containing classical adjuvant suitable for human use as shown in Figure 6.

### Determination of the preferred immunogenic composition

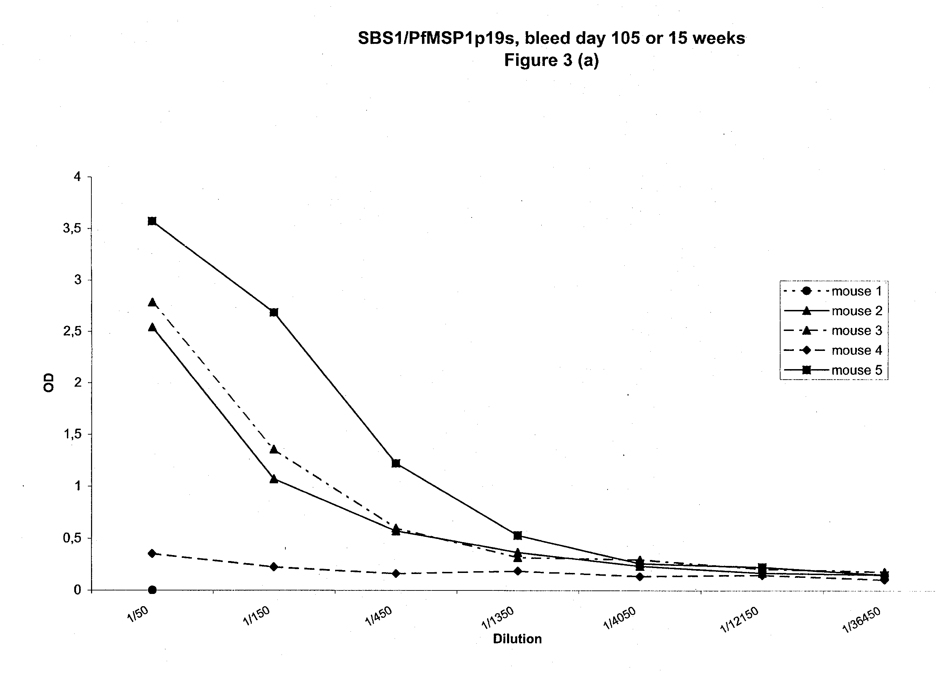

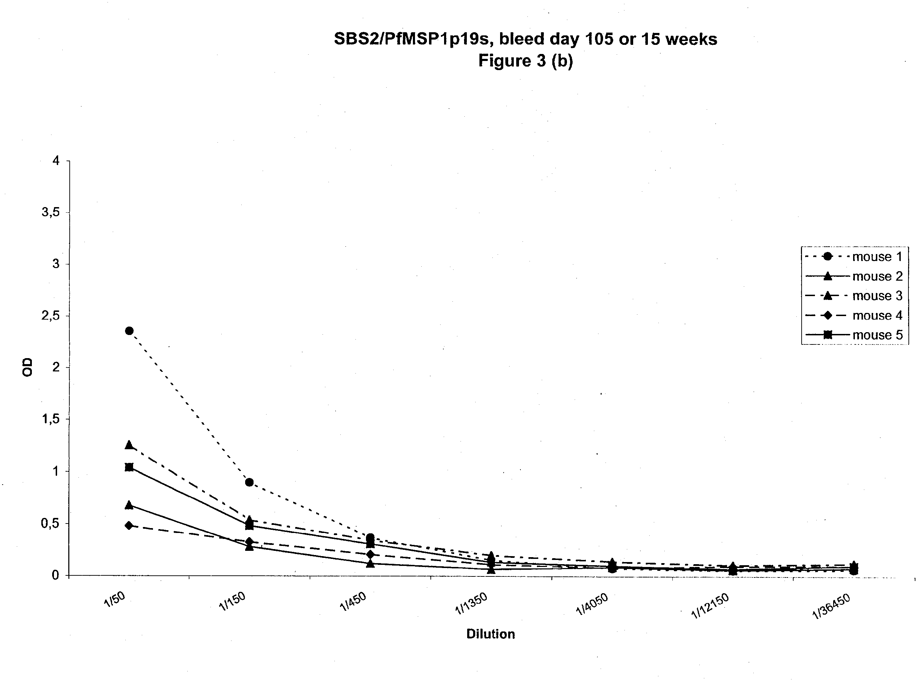

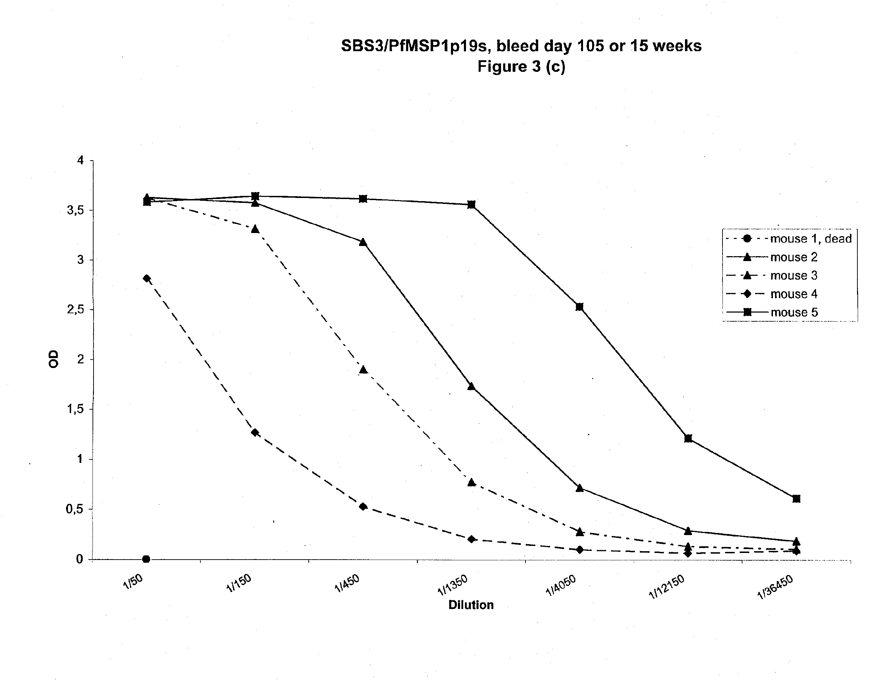

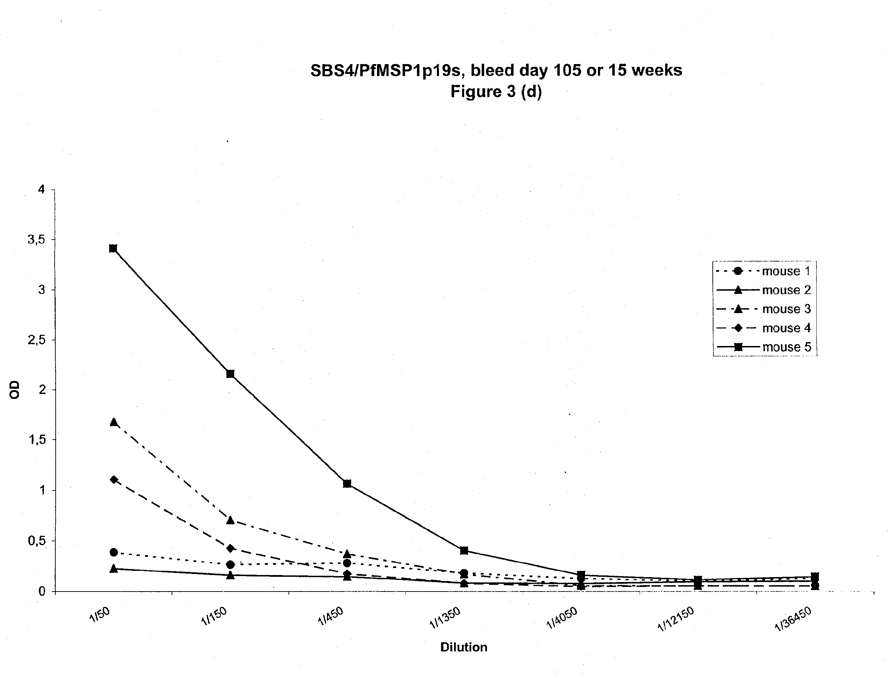

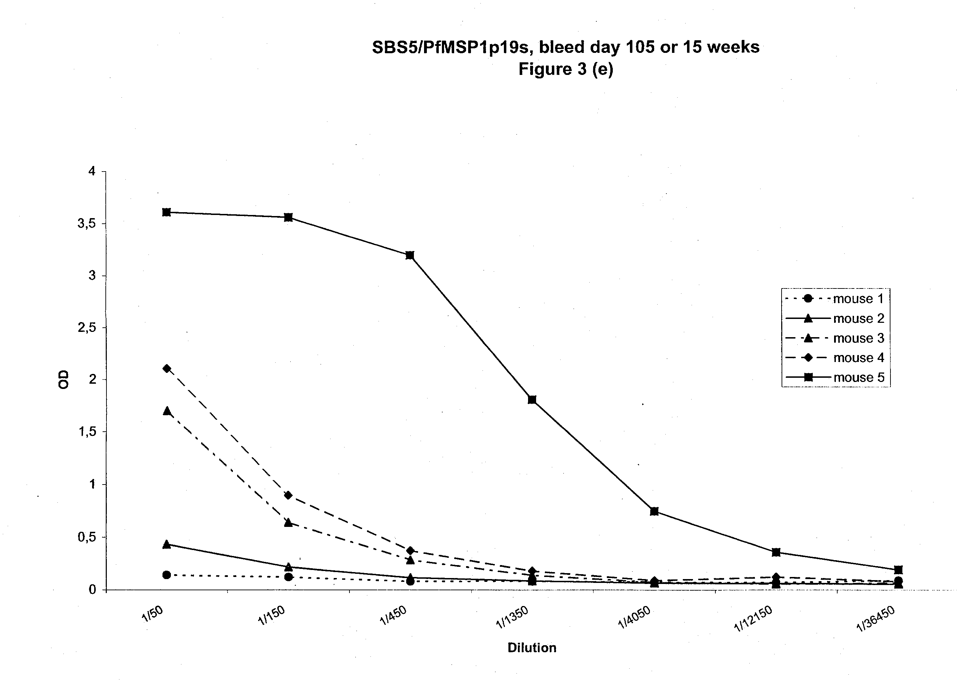

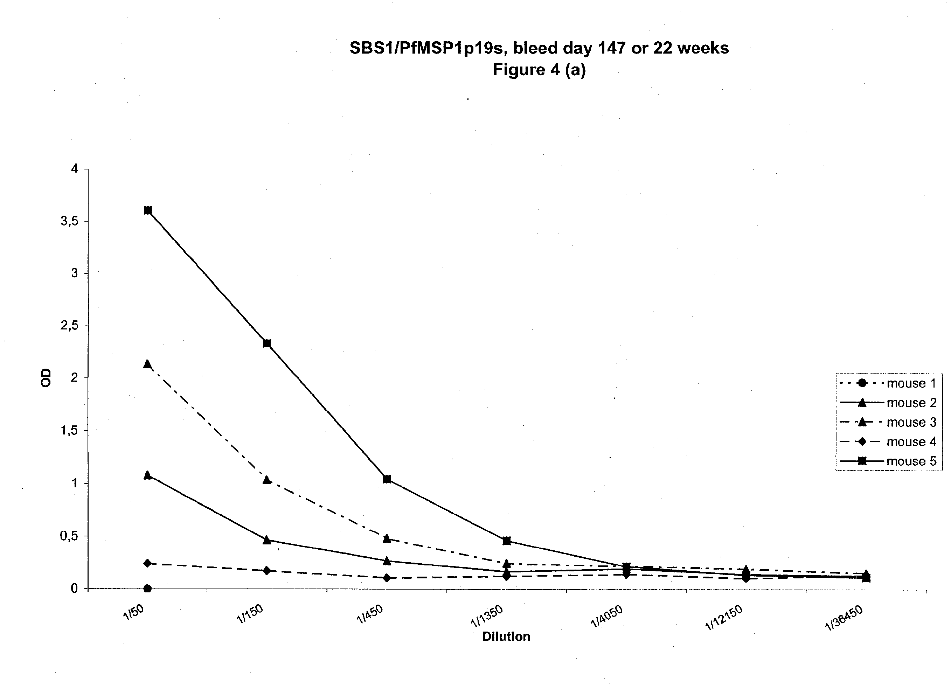

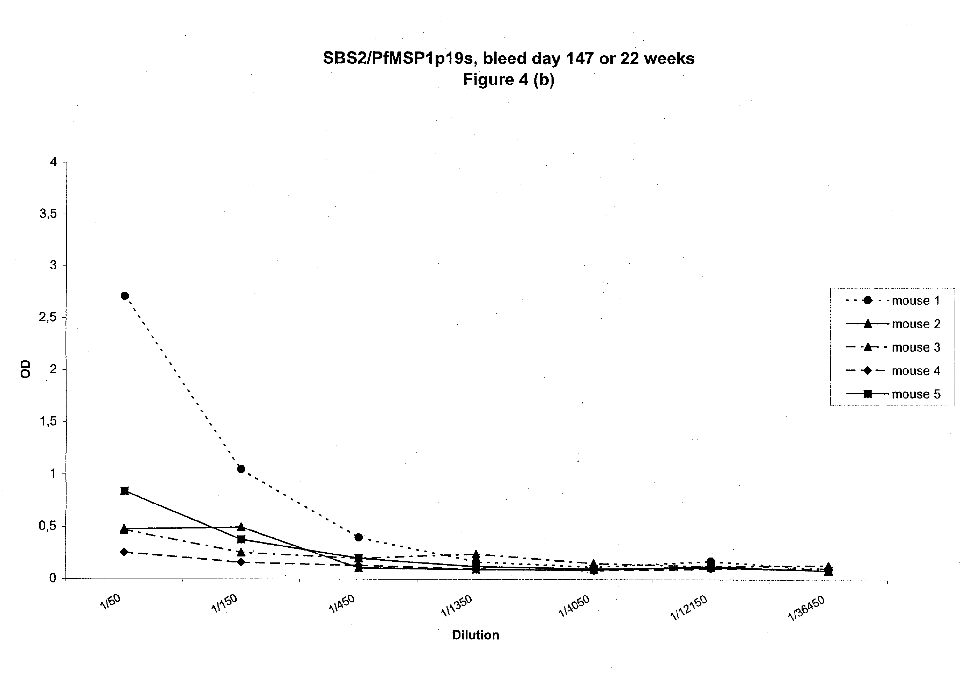

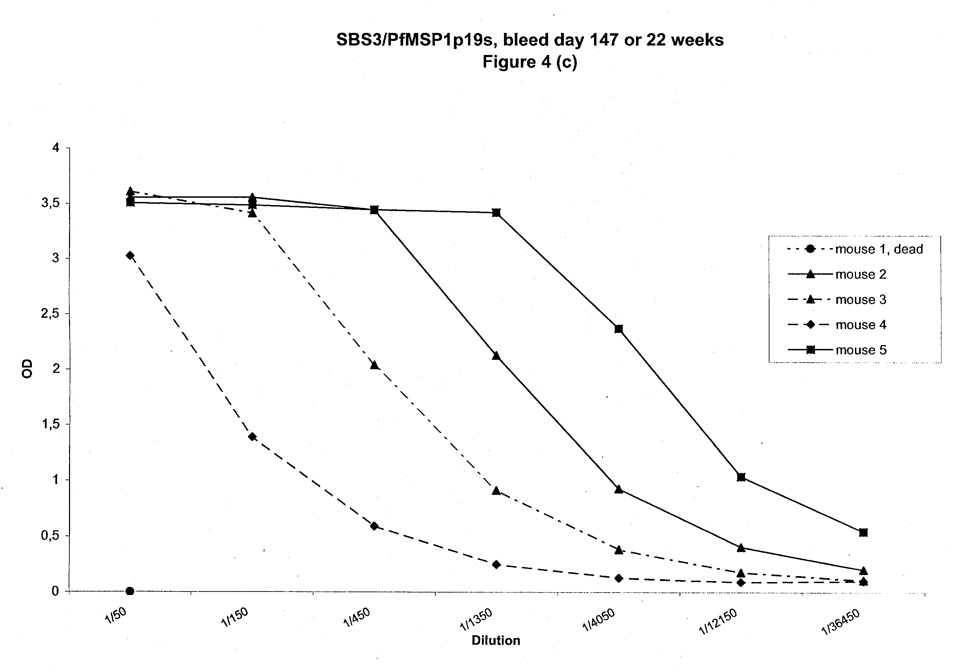

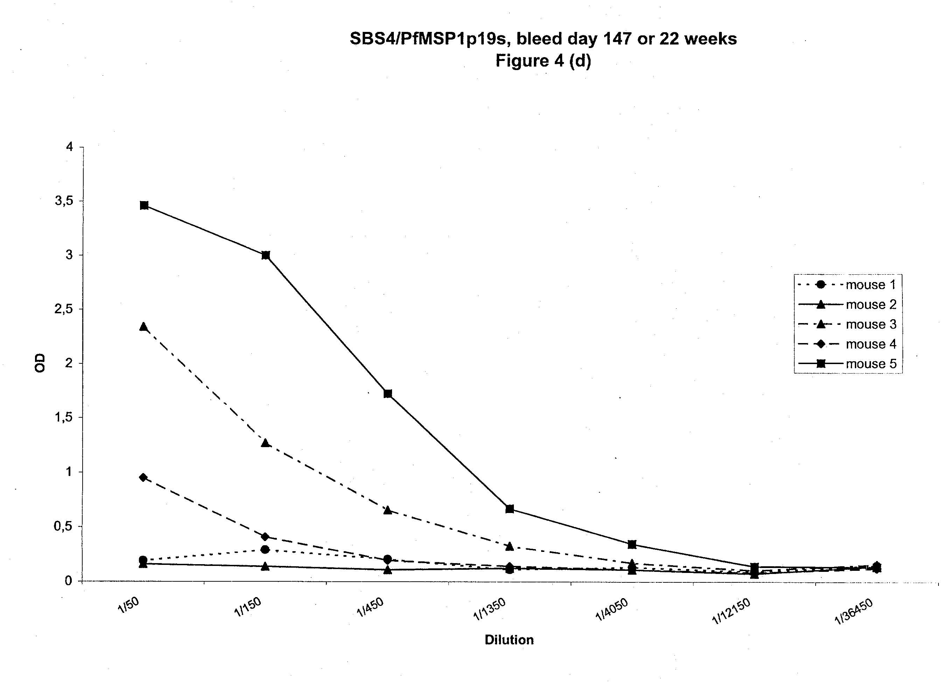

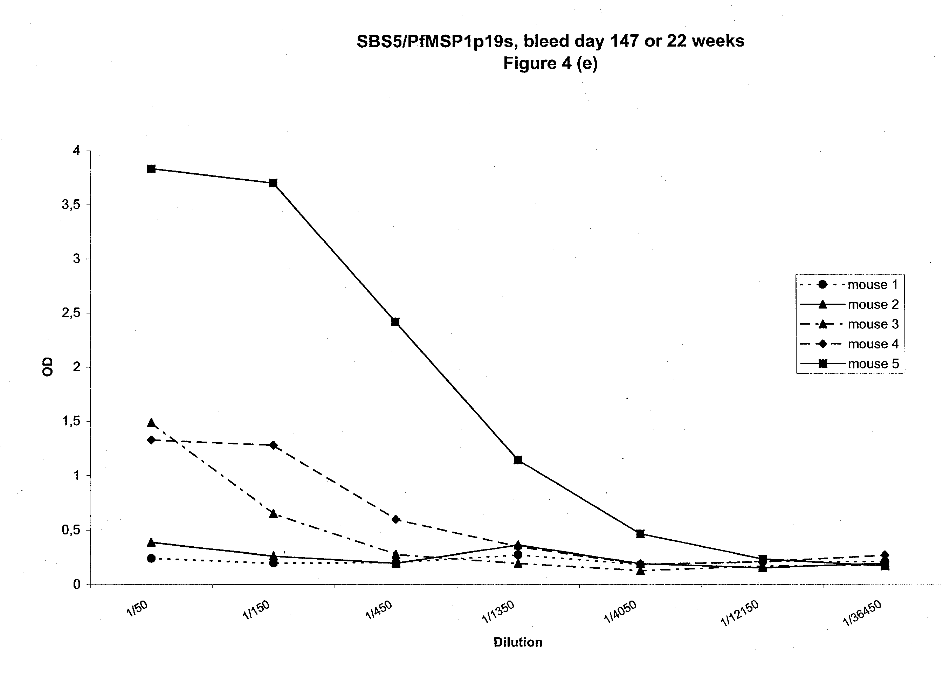

These experiments were designed to determine if compositions comprising SAIB induce a long-lived immune response and possibly an immuno-protection in a host from the first injection. An alternative specified object of these experiments is the determination of the preferred immunogenic composition tested in the present application.
Several immunogenic compositions containing SAIB were used to immunize mice. The antibody response was determined by means of an ELISA test. The immunogenic compositions used in the evaluations varied in their immunogenic potency according to the type and amount of solvents and additives associated with SAIB for a determined antigen. Nevertheless, all tested compositions in the present application have induced an immune response with a poorly immunogenic antigen, in this case, PfMSP1p19 recombinant antigen. To examine the precise potency of different SAIB compositions, several were prepared and compared in Figures 1 to 4. High antibody titers were observed for the seven compositions SBS1 to SBS7 from 3.5 weeks after the first injection. The concentration of antibody was still clearly detected 15 weeks after the first injection and without booster injection. These experiments show that the compositions containing at least an amount of SAIB from about 50% to 70% by weight of the composition have induced a long lived immune stimulation.

It is further demonstrated, in the experimental section of the present invention, that the immunogenic potency of SAIB adjuvant compositions against malaria infection can be improved by using a DLPL additive. The antibody response obtained with the composition SBS3 demonstrated that DLPL combined with SAIB has an enhancing effect on the response to the PfMSP1p19 antigen. It appears from the antibody titers determined by ELISA test that DLPL also potentiates the antibody response to the poorly immunogenic antigen. Indeed, a real synergistic effect of DLPL combined with SAIB was observed on the level and the time-periods of the antibody response. The data obtained from the ELISA analyses disclosed that the immuno-protection in the host from the first injection of SBS3 composition is the longest one among the immune responses obtained with other compositions comprising SAIB.

Therefore, the potency of the SBS3 composition inducing a long-lasting immune stimulation in the host from the first injection, is increased five to eight fold in comparison with SBS compositions.

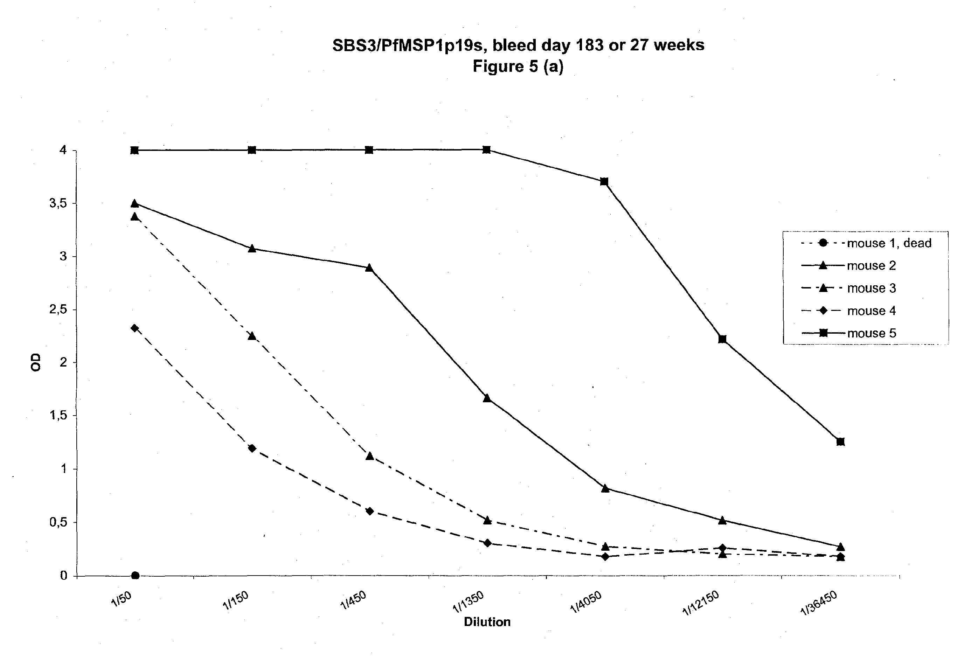

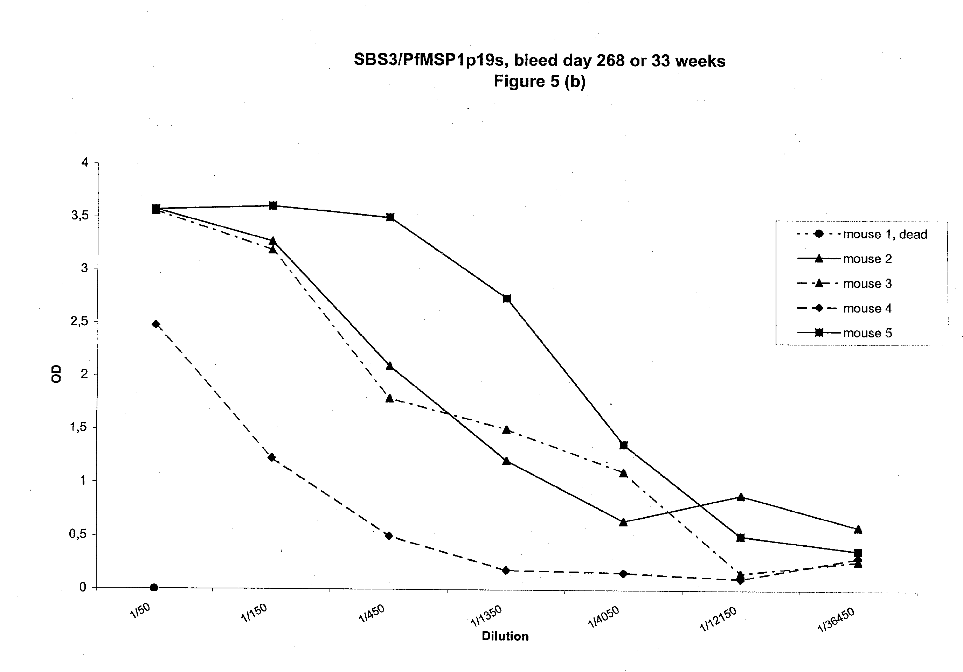

The most commonly used methods of immunization require a strict schedule of injections. The first injection is a primary immunization which induces a transitional immune response of low intensity. The second and even third injection are designed to boost the immune response to obtain a longer and stronger immune responses. The ELISA assays (Figures 5) revealed that the sera obtained from animals immunized with the SBS3 formulation recorded the highest and most durable antibody titers, since the immune response following the primary injection is detectable over 33 weeks. Therefore, this immune response can be considered equivalent to the response after booster injections. Thus, one single injection has permitted an effective immunization. These results are particularly interesting for malaria vaccines intended for developing countries where observations show that more than 30% of the patients do not return for the booster injection.

### References

**Men Y, Thomasin C, Merkle HP, Gander B, Corradin G. A single administration of tetanus toxoid in biodegradable microspheres elicits T cell and antibody responses similar or superior to those obtained with aluminum hydroxide.** Vaccine. 1995 May; 13(7):683-9.
**Langer R.** Drug delivery and targeting. Nature. 1998 ; 392 (6679 Suppl):5-10.
**Rafi-Janajreh A, Tongren JE, Kensil C, Hackett C, Candal F, Lal A, Udhayakumar V.** Influence of adjuvants in inducing immune responses to different epitopes included in a multiepitope, multivalent, multistage plasmodium falciparum candidate vaccine (FALVAC-1) in outbred mice. Experimental Parasitology 2002; 101:3-12.
**McCluskie MJ, Weeratna RD.** Novel adjuvant systems. Infectious disorders. 2001; 1:263-271.
**Lachmann PJ, Srtangeway I, Vyakarnam A, Evan G.** Raising antibodies by coupling peptides to PPD and immunizing BCG-sensitized animals. Ciba Foundation Symposium 199 1986.
**Janeway, Travers, Walport, Capra.** Immunobiology : the immune system in health and disease. Current biology , fourth edition 1999.
**Merrifield R. B.** J. Am. Chem. Soc. 1963 ; 85 : 2149-2154.
**Neimark J, Briand JP.** Development of a fully automated multichannel peptide synthesizer with integrated TFA cleavage capability. Pept Res. 1993 Jul-Aug;6(4):219-28.
**King DS, Fields CG, Fields GB.** A cleavage method which minimizes side reactions following Fmoc solid phase peptide synthesis. Int J Pept Protein Res. 1990 Sep;36(3):255-66.
**Chitarra V, Holm I, Bentley GA, Petres S, Longacre S.** The crystal structure of C-terminal merozoite surface protein 1 at 1.8 A resolution, a highly protective malaria vaccine candidate. Mol Cell. 1999 Apr;3(4):457-64.
**Gunther K, Tummler M, Arnold HH, Ridley R, Goman M, Scaife JG, Lingelbach K.** An exported protein of *Plasmodium falciparum* is synthesized as an integral membrane protein. Mol Biochem Parasitol. 1991 May;46(1 ):149-57
**Pouvelle B, Buffet PA, Lepolard C, Scherf A, Gysin J.** Cytoadhesion of *Plasmodium falciparum* ring-stage-infected erythrocytes. Nat Med. 2000 Nov;6(11):1264-8.
**Harboe M, Oettinger T, Wiker HG, Rosenkrands I, Andersen P.** Evidence for occurrence of the ESAT-6 protein in *Mycobacterium tuberculosis* and virulent *Mycobacterium bovis* and for its absence in *Mycobacterium bovis* BCG. Infect Immun. 1996 Jan;64(1):16-22.
**Berthet FX, Rasmussen PB, Rosenkrands I, Andersen P, Gicquel B.** A *Mycobacterium tuberculosis* operon encoding ESAT-6 and a novel low-molecular-mass culture filtrate protein (CFP-10). Microbiology. 1998 Nov;144 ( Pt 11):3195-203.

## Claims

1. A composition formulated to elicit or to enhance immuno-stimulation in a host against a determined antigen wherein said composition comprises:
(i) a viscous, non-polymeric, non-water soluble liquid adjuvant material; and
(ii) said determined antigen.

2. The composition according to claim 1, wherein said viscous, non-polymeric, non-water soluble liquid adjuvant material is sucrose acetate isobutyrate (SAIB).

3. The composition according to claim 2, wherein said sucrose acetate isobutyrate is present in an amount from about 99.5% to 10% by weight, relative to the weight of composition.

4. The composition according to claim 2, wherein said sucrose acetate isobutyrate is present in an amount from about 50% to 70 % by weight, relative to the weight of composition.

5. The composition according to any one of claims 1 to 4, wherein
said determined antigen is a recombinant or synthetic antigen.

6. The composition according to any one of claims 1 to 5, wherein said antigen is a malaria antigen.

7. The composition according to any one of claims 1 to 6, wherein said antigen is a malaria blood-stage antigen.

8. The composition according to any one of claims 1 to 7, wherein said antigen is selected from the group of *Plasmodium* species antigens consisting of AMA-1, EBA-175, EMP-1, GLURP, MSP-1, MSP-19, MSP-2, MSP-3, MSP-4, MSP-5, Pf35, Pf55, RAP-1, RAP-2, RESA, SERA, LSA-1, LSA-3, EXP-1, RSP-1, RSP-2, CSP-1, DBL.

9. The composition according to any one of claims 1 to 8, wherein said antigen is MSP1p19 antigen from *Plasmodium falciparum* (PfMSP1p19).

10. The composition according to any one of claims 1 to 9, wherein said antigen is a freeze-dried antigen or an able freeze-dried antigen.

11. The composition according to any one of claims 1 to 10, wherein said antigen is soluble in said viscous, non-polymeric, non-water soluble liquid adjuvant material.

12. A composition suitable for *in vivo* administration in a host, comprising a mixture of:
(i) a composition according to anyone of claims 1 to 11;
(ii) at least one pharmaceutically acceptable solvent which, when added to the viscous, non-polymeric, non-water soluble liquid adjuvant material, provides a low viscosity liquid composition.

13. The composition according to claim 12, wherein said solvent or solvents are selected from the group consisting of ethanol, ethyl lactate, propylene carbonate, glycofurol, N-methylpyrrolidone, 2-pyrrolidone, propylene glycol, acetone, methyl acetate, ethyl acetate, methyl ethyl ketone, benzyl alcohol, benzyl benzoate, benzoyl benzoate, triacetin, dimethylformamide, dimethylsulfoxide, tetrahydrofuran, aprolactam, decylmethylsulfoxide, oleic acid, triglyceride and 1-dodecylazacycloheptan-2-one.

14. The composition according to claim 13, wherein said solvent(s) is present in an amount from about 5% to 55% by weight, relative to the weight of composition.

15. The composition according to claim 13, wherein said solvent(s) is present in an amount from about 30% to 50% by weight, relative to the weight of composition.

16. The composition according to any one of claims 12 to 15, wherein the concentration of the mixture solvent(s) / viscous, non-polymeric, non-water soluble liquid adjuvant material is constant after addition of said antigen according to claim 10.

17. The composition according to anyone of claims 1 to 16, which further comprises additives.

18. The composition according to claim 17, wherein said additives are biodegradable polymers selected from the group consisting of poly(lactide), poly(lactide-co-glycolide), poly(glycolide), poly(caprolactone), polyamides, polyanhydrides, polyamino acids, polyorthoesters, polycyanoacrylates, poly(phosphazines), poly(phosphoesters), polyesteramides, polydioxanones, polyacetals, polyketals, polycarbonates, polyorthocarbonates, degradable polyurethanes, polydroxybuty-ates, polyhydroxyvalerates, polyalkylene oxalates, polyalkylene succinates, poly(malic acic), chitin, chitosan and copolymers, terpolymersoxidized cellulose.

19. The composition according to claim 18, wherein said biodegradable polymer is poly(DL-lactide) (DLPL).

20. The composition according to claim 17, wherein said additives are adjuvants.

21. The composition according to claim 20, wherein said adjuvants are selected from the group consisting of Alum, CpG DNA, various cytokines.

22. The composition according to claim 1, wherein it comprises sucrose acetate isobutyrate and N-methylpyrrolidone in a ratio of about 70:30 by weight, about 10% DLPL and 20µg of PfMSP1p19 antigen.

23. The composition according to any one of claims 1 and 22 which is suitable for topical, systemic or parenteral administration.

24. The composition according to claim 23, wherein said systemic administration consists in mucosal, oral, rectal, vaginal or nasal administration.

25. The composition according to claim 23, wherein said parenteral administration consists in intradermal, intravenous, subcutaneous, intramuscular or intraperitoneal administration.

26. The composition according to any one of claims 1 to 25 which is suitable for eliciting or enhancing an immune response in an animal, or a human host.

27. The composition according to any one of claims 1 to 26 which is suitable for eliciting an immuno-protection in a host after the first administration.

28. A kit comprising a mixture of:
(i) a composition according to any one of claims 1 to 11;
(ii) a solvent as defined in anyone of claims 12 to 16.

29. The kit according to claim 28, which further comprises additives as defined in anyone of claims 18 to 21.

30. Use of a viscous, non-polymeric, non-water soluble liquid adjuvant material for the manufacture of an adjuvant for immuno-stimulation.

31. Use of the viscous, non-polymeric, non-water soluble liquid adjuvant material according to claim 30 for the manufacture of an immunogenic composition comprising a recombinant or a synthetic antigen as active principle.

32. Use according to claim 31 wherein the immunogenic composition contains at least one malaria antigen.
